# EUROPEAN PATENT SPECIFICATION

(11) **EP 1 151 302 B1**
(45) Date of publication and mention of the grant of the patent: **31.03.2010**
(21) Application number: 00911743.3
(22) Date of filing: 09.02.2000
(51) Int. Cl.: G01N 33/543

(54) **INTRABEAD SCREENING METHODS AND COMPOSITIONS**
SCREENINGVERFAHREN MIT PORÖSEN MIKROKÜGELCHEN UND ZUSAMMENSETZUNGEN
METHODES ET COMPOSITIONS DE CRIBLAGE INTRABILLE

(30) Priority: 09.02.1999 US 119343 P
(43) Date of publication of application: 07.11.2001
(73) Proprietor: Illumina, Inc., San Diego, California 92121-1975 (US)
(72) Inventor: CZARNIK, Anthony, W., San Diego, CA 92127 (US); WALT, David, R., Lexington, MA 02173 (US); STUELPNAGEL, John, R., Encinitas, CA 92024 (US)
(74) Representative: Kiddle, Simon John
(86) International application number: PCT/US2000/003370
(87) International publication number: WO 2000/048000

(56) References cited:
- WO-A-94/02515
- WO-A-96/36436
- WO-A-98/08092
- WO-A-98/40726

## Description

### FIELD OF THE INVENTION

The invention relates to the use of porous beads to screen candidate bioactive agents for the ability to interact or effect a target substance. The screen is performed in the interior and exterior surface of the bead, allowing extremely small sample sizes and concentration of reagents, while retarding evaporation.

### BACKGROUND OF THE INVENTION

Traditional approaches to identify and characterize new and useful drug candidates include the isolation of natural products or synthetic preparation, followed by testing against either known or unknown targets. See for example WO 94/24314, Gallop et al., J. Med. Chem. 37(9):1233 (1994); Gallop et al., J. Med. Chem. 37(10):1385 (1994); Ellman, Acc. Chem. Res. 29:132 (1996); Gordon et al., E. J. Med. Chem. 30:388s (1994); Gordon et al., Acc. Chem. Res. 29:144 (1996); WO 95/12608.

The screening of these libraries is done in a variety of ways. One approach involves attachment to beads and visualization with dyes; see Neslter et al., Bioorg. Med. Chem. Lett. 6(12):1327 (1996). Another approach has utilized beads and fluorescence activated cell sorting (FACS); see Needles et al., PNAS USA 90:10700 (1993), and Vetter et al., Bioconjugate Chem. 6:319 (1995).

In addition, there are a number of assays and sensors for the detection of the presence and/or concentration of specific substances in fluids and gases. Many of these rely on specific ligand/antiligand reactions as the mechanism of detection. That is, pairs of substances (i.e. the binding pairs or ligand/antiligands) are known to bind to each other, while binding little or not at all to other substances. This has been the focus of a number of techniques that utilize these binding pairs for the detection of the complexes. These generally are done by labeling one component of the complex in some way, so as to make the entire complex detectable, using, for example, radioisotopes, fluorescent and other optically active molecules, enzymes, etc.

Of particular use in these sensors are detection mechanisms utilizing luminescence. Recently, the use of optical fibers and optical fiber strands in combination with light absorbing dyes for chemical analytical determinations has undergone rapid development, particularly within the last decade. The use of optical fibers for such purposes and techniques is described by Milanovich et al., "Novel Optical Fiber Techniques For Medical Application", Proceedings of the SPIE 28th Annual International Technical Symposium On Optics and Electro-Optics, Volume 494, 1980; Seitz, W.R., "Chemical Sensors Based On Immobilized Indicators and Fiber Optics" in C.R.C. Critical Reviews In Analytical Chemistry, Vol. 19, 1988, pp. 135-173; Wolfbeis, O.S., "Fiber Optical Fluorosensors In Analytical Chemistry" in Molecular Luminescence Spectroscopy, Methods and Applications (S. G. Schulman, editor), Wiley & Sons, New York (1988); Angel, S.M., Spectroscopy 2 (4):38 (1987); Walt, et al., "Chemical Sensors and Microinstrumentation", ACS Symposium Series, Vol. 403, 1989, p. 252, and Wolfbeis, O.S., Fiber Optic Chemical Sensors, Ed. CRC Press, Boca Raton, FL, 1991, 2nd Volume.

When using an optical fiber in an *in vitro*/*in vivo* sensor, one or more light absorbing dyes are located near its distal end. Typically, light from an appropriate source is used to illuminate the dyes through the fiber's proximal end. The light propagates along the length of the optical fiber; and a portion of this propagated light exits the distal end and is absorbed by the dyes. The light absorbing dye may or may not be immobilized; may or may not be directly attached to the optical fiber itself; may or may not be suspended in a fluid sample containing one or more analytes of interest; and may or may not be retainable for subsequent use in a second optical determination.

Once the light has been absorbed by the dye, some light of varying wavelength and intensity returns, conveyed through either the same fiber or collection fiber(s) to a detection system where it is observed and measured. The interactions between the light conveyed by the optical fiber and the properties of the light absorbing dye provide an optical basis for both qualitative and quantitative determinations.

Of the many different classes of light absorbing dyes which conventionally are employed with bundles of fiber strands and optical fibers for different analytical purposes are those more common compositions that emit light after absorption termed "fluorophores" and those which absorb light and internally convert the absorbed light to heat, rather than emit it as light, termed "chromophores."

Fluorescence is a physical phenomenon based upon the ability of some molecules to absorb light (photons) at specified wavelengths and then emit light of a longer wavelength and at a lower energy. Substances able to fluoresce share a number of common characteristics: the ability to absorb light energy at one wavelength λₑₓ; reach an excited energy state; and subsequently emit light at another light wavelength, λₑₘ. The absorption and fluorescence emission spectra are individual for each fluorophore and are often graphically represented as two separate curves that are slightly overlapping. The same fluorescence emission spectrum is generally observed irrespective of the wavelength of the exciting light and, accordingly, the wavelength and energy of the exciting light may be varied within limits; but the light emitted by the fluorophore will always provide the same emission spectrum under a given set of conditions. Finally, the strength of the fluorescence signal may be measured as the quantum yield of light emitted. The fluorescence quantum yield is the ratio of the number of photons emitted in comparison to the number of photons initially absorbed by the fluorophore. For more detailed information regarding each of these characteristics, the following references are recommended: Lakowicz, J. R., Principles of Fluorescence Spectroscopy, Plenum Press, New York, 1983; Freifelder, D., Physical Biochemistry, second edition, W. H. Freeman and Company, New York, 1982; "Molecular Luminescence Spectroscopy Methods and Applications: Part I" (S.G. Schulman, editor) in Chemical Analysis, vol. 77, Wiley & Sons, Inc., 1985; The Theory of Luminescence, Stepanov and Gribkovskii, Iliffe Books, Ltd., London, 1968.

In comparison, substances which absorb light and do not fluoresce usually convert the light into heat or kinetic energy. The ability to internally convert the absorbed light identifies the dye as a "chromophore." Dyes which absorb light energy as chromophores do so at individual wavelengths of energy and are characterized by a distinctive molar absorption coefficient at that wavelength. Chemical analysis employing fiber optic strands and absorption spectroscopy using visible and ultraviolet light wavelengths in combination with the absorption coefficient allow for the determination of concentration for specific analyses of interest by spectral measurement. The most common use of absorbance measurement via optical fibers is to determine concentration which is calculated in accordance with Beers' law; accordingly, at a single absorbance wavelength, the greater the quantity of the composition which absorbs light energy at a given wavelength, the greater the optical density for the sample. In this way, the total quantity of light absorbed directly correlates with the quantity of the composition in the sample.

Many of the recent improvements employing optical fiber sensors in both qualitative and quantitative analytical determinations concern the desirability of depositing and/or immobilizing various light absorbing dyes at the distal end of the optical fiber. In this manner, a variety of different optical fiber chemical sensors and methods have been reported for specific analytical determinations and applications such as pH measurement, oxygen detection, and carbon dioxide analyses. These developments are exemplified by the following publications: Freeman, et al., Anal Chem. 53:98 (1983); Lippitsch et al., Anal. Chem. Acta. 205:1, (1988); Wolfbeis et al., Anal. Chem. 60:2028 (1988); Jordan, et al., Anal. Chem. 59:437 (1987); Lubbers et al., Sens. Actuators 1983; Munkholm et al., Talanta 35:109 (1988); Munkholm et al., Anal. Chem. 58:1427 (1986); Seitz, W. R., Anal. Chem. 56:16A-34A (1984); Peterson, et al., Anal. Chem. 52:864 (1980): Saari, et al., Anal. Chem. 54:821 (1982); Saari, et al., Anal. Chem. 55:667 (1983); Zhujun et al., Anal. Chem. Acta. 160:47 (1984); Schwab, et al., Anal. Chem. 56:2199 (1984); Wolfbeis, O.S., "Fiber Optic Chemical Sensors", Ed. CRC Press, Boca Raton, FL, 1991, 2nd Volume; and Pantano, P., Walt, D.R., Anal. Chem., 481A-487A, Vol. 67, (1995).

More recently, fiber optic sensors have been constructed that permit the use of multiple dyes with a single, discrete fiber optic bundle. U.S. Pat. Nos. 5,244,636 and 5,250,264 to Walt, et al. disclose systems for affixing multiple, different dyes on the distal end of the bundle, the teachings of each of these patents being incorporated herein by this reference. The disclosed configurations enable separate optical fibers of the bundle to optically access individual dyes. This avoids the problem of deconvolving the separate signals in the returning light from each dye, which arises when the signals from two or more dyes are combined, each dye being sensitive to a different analyte, and there is significant overlap in the dyes' emission spectra.

U.S. Patent No. 6,023,540 describes array compositions that utilize microspheres or beads on a terminal end of a fiber optic bundle, with each individual fiber comprising a bead containing an optical signature. Since the beads go down randomly, a unique optical signature is needed to "decode" the array; i.e. after the array is made, a correlation of the location of an individual site on the array with the bead or bioactive agent at that particular site can be made. This means that the beads may be randomly distributed on the array, a fast and inexpensive process as compared to either the *in situ* synthesis or spotting techniques of the prior art. Once the array is loaded with the beads, the array can be decoded, or can be used, with full or partial decoding occuring after testing, as is described in U.S.S.N.s 60/090,473 and 09/189,543 (priority applications for WO 99/67641).

International Publication No. WO94/02515 describes arrays comprising oligomer libraries. In particular, this publication describes arrays comprising a population of beads surrounded by a substrate. Oligomers are attached to the beads by cleavable linkers. Cleavage of the linkers facilitates the removal of the oligomer from the beads. The surrounding substrate allows the oligomers to diffuse away from the beads but limits the mixing of oligomers cleaved from neighboring beads in the population.

International Publication No. WO98/08092 describes processes for making and using bead-based arrays containing combinatorial libraries. In particular, the arrays comprise reversibly attached beads of uniform size. In addition to being reversibly attached to the array, the beads are attached to the library compounds via a cleavable linker. As the beads are detached from the array, they are drawn into capillaries where they are retained. Cleavage of the linker permits the compounds to diffuse through the capillary away from the retained bead to a location where they can be further analyzed.

Accordingly, it is an object of the present invention to provide compositions and methods for the screening of candidate bioactive agents for their ability to interact or effect a target analyte.

### SUMMARY OF THE INVENTION

In accordance with the objects outlined above, the present invention provides an array composition comprising a substrate with a surface comprising discrete sites; and a population of microspheres comprising at least a first and a second subpopulation. Each subpopulation comprises a candidate bioactive agent and an identifier moiety. Said microspheres are porous and are associated with or attached to the discrete sites on the surface of the substrate and said candidate bioactive agents are linked to said microspheres via a scissile linkage.

In an additional aspect, the invention provides array compositions comprising a substrate with a surface comprising discrete sites: and a population of porous microspheres comprising at least a first and a second subpopulation, wherein said microspheres are distributed on said surface. The microspheres are associated with or attached to the discrete sites on the surface of the substrate, and said first and said second subpopulation each comprise the same candidate bioactive agent linked to said microspheres via a scissile linkage. The amount of candidate agent on the first subpopulation is different from the amount of candidate agent on the second subpopulation, such that different concentrations of candidate agents can be assayed.

In a further aspect, the invention provides assay methods for detecting the binding of a candidate bioactive agent to a target analyte comprising adding a solution comprising at least one target analyte to a plurality of porous microspheres, wherein said microspheres are associated with or attached to the discrete sites on a surface of a substrate or are present in a diffusion-retarding solvent, and wherein said microspheres comprise at least a first and a second subpopulation, wherein each subpopulation comprises a candidate bioactive agent linked to the microspheres via a scissile linkage and an identifier moiety.

The scissile linkage is then cleaved, and the binding of at least one candidate bioactive agent to the target analyte is detected.

### DETAILED DESCRIPTION OF THE INVENTION

The present invention is directed to "in-bead" or "intrabead" screening, wherein assays, particularly binding assays, are run within and on a porous bead. That is, candidate bioactive agents are synthesized on a porous bead, resulting in the candidate agents being attached, frequently via a scissile linkage, to the internal and external surface of a bead. As is more fully described below, these beads can be randomly distributed at discrete sites on a surface, for example a terminus of a fiber optic bundle, such that extremely large numbers of assays can be simultaneously run. The assay reagents can then be added, such as buffers, co-factors, metal ions and otherassay reagents, and the target analyte for which binding is being evaluated, and the candidate bioactive agent is cleaved from the bead, allowing free diffusion through the bead and interaction with the target. Detection of binding is then accomplished in a variety of ways, as is more fully outlined below.

Thus the present invention has a number of important advantages, including the use of very low amounts of reagents, a distinct spatial localization of the assays, decreased propensity to diffusion and decreased rates of solvent evaporation. In addition, as is more fully outlined below, by varying the amount of candidate bioactive agent attached to each bead, the concentration of the candidate bioactive agent can be varied, allowing the simultaneous determination of concentration effects. This is quite significant, as it allows the determination of kinetic parameters such as Kₘ and Kᵢ and also avoids subsequent scale-up, including resynthesis of the agents and reassays.

Accordingly, the present invention provides array compositions. By "array" herein is meant a plurality of candidate agents in an array format; the size of the array will depend on the composition and end use of the array. Arrays containing from about 2 different bioactive agents (i.e. different beads) to many millions can be made, with very large fiber optic arrays being possible. Generally, the array will comprise from two to as many as a billion or more, depending on the size of the beads and the substrate, as well as the end use of the array, thus very high density, high density, moderate density, low density and very low density arrays may be made. Preferred ranges for very high density arrays (all numbers are per cm²) are from about 10,000,000 to about 2,000,000,000, with from about 100,000,000 to about 1,000,000,000 being preferred. High density arrays range from about 100,000 to about 10,000,000, with from about 1,000,000 to about 5,000,000 being particularly preferred. Moderate density arrays range from about 10,000 to about 100,000 being particularly preferred, and from about 20,000 to about 50,000 being especially preferred. Low density arrays are generally less than 10,000, with from about 1,000 to about 5,000 being preferred. Very low density arrays are less than 1,000, with from about 10 to about 1000 being preferred, and from about 100 to about 500 being particularly preferred. In some embodiments, the compositions of the invention may not be in array format; that is, for some embodiments, compositions comprising a single bioactive agent may be made as well. In addition, in some arrays, multiple substrates may be used, either of different or identical compositions. Thus for example, large arrays may comprise a plurality of smaller substrates.

In addition, one advantage of the present compositions is that particularly through the use of fiber optic technology, extremely high density arrays can be made. Thus for example, because beads of 200 µm or less (with beads of 200 nm possible) can be used, and very small fibers are known, it is possible to have as many as 40,000 - 50,000 or more (in some instances, 1 million) different fibers and beads in a 1 mm² fiber optic bundle, with densities of greater than 15,000,000 individual beads and fibers (again, in some instances as many as 25-50 million) per 0.5 cm² obtainable.

The arrays comprise a substrate to which the beads are associated. By "substrate" or "solid support" or other grammatical equivalents herein is meant any material that can be modified to contain discrete individual sites appropriate for the attachment or association of beads and is amenable to at least one detection method. As will be appreciated by those in the art, the number of possible substrates is very large. Possible substrates include, but are not limited to, glass and modified or functionalized glass, plastics (including acrylics, polystyrene and copolymers of styrene and other materials, polypropylene, polyethylene, polybutylene, polyurethanes, Teflon®, etc.), polysaccharides, nylon or nitrocellulose, resins, silica or silica-based materials including silicon and modified silicon, carbon, metals, inorganic glasses, plastics, optical fiber bundles, and a variety of other polymers. In general, the substrates allow optical detection and do not themselves appreciably fluorescese.

Generally the substrate is flat (planar), although as will be appreciated by those in the art, other configurations of substrates may be used as well; for example, three dimensional configurations can be used, for example by embedding the beads in a porous block of plastic that allows sample access to the beads and using a confocal microscope for detection. Similarly, the beads may be placed on the inside surface of a tube, for flow-through sample analysis to minimize sample volume. Preferred substrates include optical fiber bundles as discussed below, and flat planar substrates such as glass, polystyrene and other plastics and acrylics.

In a preferred embodiment, the substrate is an optical fiber bundle or array, as is generally described in WO 9918434 and US 6200737, WO 9840726, and WO 9850782. Preferred embodiments utilize preformed unitary fiber optic arrays. By "preformed unitary fiber optic array" herein is meant an array of discrete individual fiber optic strands that are co-axially disposed and joined along their lengths. The fiber strands are generally individually clad. However, one thing that distinguishes a preformed unitary array from other fiber optic formats is that the fibers are not individually physically manipulatable; that is, one strand generally cannot be physically separated at any point along its length from another fiber strand.

At least one surface of the substrate is modified to contain discrete, individual sites for later association of microspheres. These sites may comprise physically altered sites, i.e. physical configurations such as wells or small depressions in the substrate that can retain the beads, such that a microsphere can rest in the well, or the use of other forces (magnetic or compressive), or chemically altered or active sites, such as chemically functionalized sites, electrostatically altered sites, hydrophobically/ hydrophilically functionalized sites, spots of adhesive, etc.

The sites may be a pattern, i.e. a regular design or configuration, or randomly distributed. A preferred embodiment utilizes a regular pattern of sites such that the sites may be addressed in the X-Y coordinate plane. "Pattern" in this sense includes a repeating unit cell, preferably one that allows a high density of beads on the substrate. However, it should be noted that these sites may not be discrete sites. That is, it is possible to use a uniform surface of adhesive or chemical functionalities, for example, that allows the attachment of beads at any position. That is, the surface of the substrate is modified to allow attachment of the microspheres at individual sites, whether or not those sites are contiguous or non-contiguous with other sites. Thus, the surface of the substrate may be modified such that discrete sites are formed that can only have a single associated bead, or alternatively, the surface of the substrate is modified and beads may go down anywhere, but they end up at discrete sites.

In a preferred embodiment, the surface of the substrate is modified to contain wells, i.e. depressions in the surface of the substrate. This may be done as is generally known in the art using a variety of techniques, including, but not limited to, photolithography, stamping techniques, laser ablation, molding techniques and microetching techniques. As will be appreciated by those in the art, the technique used will depend on the composition and shape of the substrate. Thus, for example, for plastic substrates, the substrate may be molded or stamped during manufacture to produce the wells; fiber optic bundles may be chemically etched.

In a preferred embodiment, physical alterations are made in a surface of the substrate to produce the sites. In a preferred embodiment, the substrate is a fiber optic bundle and the surface of the substrate is a terminal end of the fiber bundle, as is generally described in WO 9840726. In this embodiment, wells are made in a terminal or distal end of a fiber optic bundle comprising individual fibers. In this embodiment, the cores of the individual fibers are etched, with respect to the cladding, such that small wells or depressions are formed at one end of the fibers. The methods used will depend on the composition of the substrate, with chemical etching being preferred for fiber optic bundles, for example using ammonium fluoride and hydrofluoric acid. The required depth of the wells will depend, in part, on the size of the beads to be added to the wells.

Generally in this embodiment, the microspheres are non-covalently associated in the wells, although the wells may additionally be chemically functionalized as is generally described below, cross-linking agents may be used, or a physical barrier may be used, i.e. a film or membrane over the beads.

In a preferred embodiment, the beads are attached non-covalently through non-specific interactions such as Van der Waals forces.

In a preferred embodiment, the surface of the substrate is modified to contain chemically modified sites that can be used to attach, either covalently or non-covalently, the microspheres of the invention to the discrete sites or locations on the substrate. "Chemically modified sites" in this context includes, but is not limited to, the addition of a pattern of chemical functional groups including amino groups, carboxy groups, oxo groups and thiol groups, that can be used to covalently attach microspheres, which generally also contain corresponding reactive functional groups; the addition of a pattern of adhesive that can be used to bind the microspheres (either by prior chemical functionalization for the addition of the adhesive or direct addition of the adhesive); the addition of a pattern of charged groups (similar to the chemical functionalities) for the electrostatic attachment of the microspheres, i.e. when the microspheres comprise charged groups opposite to the sites; the addition of a pattern of chemical functional groups that renders the sites differentially hydrophobic or hydrophilic, such that the addition of similarly hydrophobic or hydrophilic microspheres under suitable experimental conditions will result in association of the microspheres to the sites on the basis of hydroaffinity. For example, the use of hydrophobic sites with hydrophobic beads, in an aqueous system, drives the association of the beads preferentially onto the sites. As outlined above, "pattern" in this sense includes the use of a uniform treatment of the surface to allow attachment of the beads at discrete sites, as well as treatment of the surface resulting in discrete sites. As will be appreciated by those in the art, this may be accomplished in a variety of ways.

The compositions of the invention further comprise a population of microspheres. By "population" herein is meant a plurality of beads as outlined above for arrays. Within the population are separate subpopulations, which can be a single microsphere or multiple identical microspheres. That is, in some embodiments, as is more fully outlined below, the array may contain only a single bead for each bioactive agent; preferred embodiments utilize a plurality of beads of each type.

By "microspheres" or "beads" or "particles" or grammatical equivalents herein is meant small discrete particles. The composition of the beads will vary, depending on the class of bioactive agent and the method of synthesis. Suitable bead compositions include those used in peptide, nucleic acid and organic moiety synthesis, including, but not limited to, plastics, ceramics, glass, polystyrene, methylstyrene, acrylic polymers including polyacrylimide, paramagnetic materials, thoria sol, carbon graphited, titanium dioxide, latex or cross-linked dextrans such as Sepharose, cellulose, nylon, cross-linked micelles and teflon may all be used. "Microsphere Detection Guide" from Bangs Laboratories, Fishers IN is a helpful guide.

The beads are porous. That is, the interior of the bead is solvent accessible, with beads that are up to roughly 80% solvent (e.g. water) being preferred. Preferred embodiments utilize beads that have large internal surface areas as compared to the external surface of the bead. In addition, the pores are generally large enough to easily accomodate facile diffusion of biological molecules, particularly proteins and nucleic acids. Porous beads are generally made as is known in the art and generally result from differences in cross-linking and solvent conditions during formation.

The beads need not be spherical; irregular particles may be used. The bead sizes range from nanometers, i.e. 100 nm, to millimeters, i.e. 1 mm, with beads from about 0.2 micron to about 200 microns being preferred, and from about 0.5 to about 5 micron being particularly preferred, although in some embodiments smaller beads may be used.

It should be noted that a key component of the invention is the use of a substrate/bead pairing that allows the association or attachment of the beads at discrete sites on the surface of the substrate, such that the beads do not move during the course of the assay.

Each microsphere comprises at least one bioactive agent, although as will be appreciated by those in the art, there may be some microspheres which do not contain a bioactive agent, depending on the synthetic methods. Similarly, although not preferred, are beads comprising more than one bioactive agent. By "candidate bioactive agent" or "bioactive agent" or "chemical functionality" or "binding ligand" herein is meant as used herein describes any molecule, e.g., protein, oligopeptide, small organic molecule, coordination complex, polysaccharide, polynucleotide, etc. that can be attached to the microspheres of the invention. It should be understood that the compositions of the invention have two primary uses. In a preferred embodiment, as is more fully outlined below, the compositions are used to detect the binding of a candidate bioactive agent to a particular target analyte; for example, for screening of enzyme inhibitors or protein-protein interactions.

Bioactive agents encompass numerous chemical classes, though typically they are organic molecules, preferably small organic compounds having a molecular weight of more than 100 and less than about 2,500 daltons. Bioactive agents comprise functional groups necessary for structural interaction with proteins, particularly hydrogen bonding, and typically include at least an amine, carbonyl, hydroxyl or carboxyl group, preferably at least two of the functional chemical groups. The bioactive agents often comprise cyclical carbon or heterocyclic structures and/or aromatic or polyaromatic structures substituted with one or more of the above functional groups. Bioactive agents are also found among biomolecules including peptides, nucleic acids, saccharides, fatty acids, steroids, purines, pyrimidines, derivatives, structural analogs or combinations thereof. Particularly preferred are nucleic acids and proteins.

Bioactive agents can be obtained from a wide variety of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of bacterial, fungal, plant and animal extracts are available or readily produced. Additionally, natural or synthetically produced libraries and compounds are readily modified through conventional chemical, physical and biochemical means. Known pharmacological agents may be subjected to directed or random chemical modifications, such as acylation, alkylation, esterification and/or amidification to produce structural analogs.

In a preferred embodiment, the bioactive agents are proteins. By "protein" herein is meant at least two covalently attached amino acids, which includes proteins, polypeptides, oligopeptides and peptides. The protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and norleucine are considered amino acids for the purposes of the invention. The side chains may be in either the (R) or the (S) configuration. In the preferred embodiment, the amino acids are in the (S) or L-configuration. If non-naturally occurring side chains are used, non-amino acid substituents may be used, for example to prevent or retard in vivo degradations.

In one preferred embodiment, the bioactive agents are naturally occurring proteins or fragments of naturally occuring proteins. Thus, for example, cellular extracts containing proteins, or random or directed digests of proteinaceous cellular extracts, may be used. In this way libraries of procaryotic and eukaryotic proteins may be made for screening in the systems described herein. Particularly preferred in this embodiment are libraries of bacterial, fungal, viral, and mammalian proteins, with the latter being preferred, and human proteins being especially preferred.

In a preferred embodiment, the bioactive agents are peptides of from about 5 to about 30 amino acids, with from about 5 to about 20 amino acids being preferred, and from about 7 to about 15 being particularly preferred. The peptides may be digests of naturally occurring proteins as is outlined above, random peptides, or "biased" random peptides. By "randomized" or grammatical equivalents herein is meant that each nucleic acid and peptide consists of essentially random nucleotides and amino acids, respectively. Since generally these random peptides (or nucleic acids, discussed below) are chemically synthesized, they may incorporate any nucleotide or amino acid at any position. The synthetic process can be designed to generate randomized proteins or nucleic acids, to allow the formation of all or most of the possible combinations over the length of the sequence, thus forming a library of randomized bioactive proteinaceous agents.

In a preferred embodiment, a library of bioactive agents are used. The library should provide a sufficiently structurally diverse population of bioactive agents to effect a probabilistically sufficient range of binding to target analytes. Accordingly, an interaction library must be large enough so that at least one of its members will have a structure that gives it affinity for the target analyte. Although it is difficult to gauge the required absolute size of an interaction library, nature provides a hint with the immune response: a diversity of 10⁷-10⁸ different antibodies provides at least one combination with sufficient affinity to interact with most potential antigens faced by an organism. Published in vitro selection techniques have also shown that a library size of 10⁷ to 10⁸ is sufficient to find structures with affinity for the target. Thus, in a preferred embodiment, at least 10⁶, preferably at least 10⁷, more preferably at least 10⁸ and most preferably at least 10⁹ different bioactive agents are simultaneously analyzed in the subject methods. Preferred methods maximize library size and diversity.

In a preferred embodiment, the library is fully randomized, with no sequence preferences or constants at any position. In a preferred embodiment, the library is biased. That is, some positions within the sequence are either held constant, or are selected from a limited number of possibilities. For example, in a preferred embodiment, the nucleotides or amino acid residues are randomized within a defined class, for example, of hydrophobic amino acids, hydrophilic residues, sterically biased (either small or large) residues, towards the creation of cysteines, for cross-linking, prolines for SH-3 domains, serines, threonines, tyrosines or histidines for phosphorylation sites, etc., or to purines, etc.

In a preferred embodiment, the bioactive agents are nucleic acids (generally called "probe nucleic acids" or "candidate probes" herein). By "nucleic acid" or "oligonucleotide" or grammatical equivalents herein means at least two nucleotides covalently linked together. A nucleic acid of the present invention will generally contain phosphodiester bonds, although in some cases, as outlined below, nucleic acid analogs are included that may have alternate backbones, comprising, for example, phosphoramide (Beaucage, et al., Tetrahedron, 49(10):1925 (1993) and references therein; Letsinger, J. Org. Chem., 35:3800 (1970); Sprinzl, et al., Eur. J. Biochem., 81:579 (1977); Letsinger, et al., Nucl. Acids Res., 14:3487 (1986); Sawai, et al., Chem. Lett., 805 (1984), Letsinger, et al., J. Am. Chem. Soc., 110:4470 (1988); and Pauwels, et al., Chemica Scripta, 26:141 (1986)), phosphorothioate (Mag, et al., Nucleic Acids Res., 19:1437 (1991); and U.S. Patent No. 5,644,048), phosphorodithioate (Briu, et al., J. Am. Chem. Soc., 111:2321 (1989)), O-methylphophoroamidite linkages (see Eckstein, Oligonucleotides and Analogues: A Practical Approach, Oxford University Press), and peptide nucleic acid backbones and linkages (see Egholm, J. Am. Chem. Soc., 114:1895 (1992); Meier, et al., Chem. Int. Ed. Engl., 31:1008 (1992); Nielsen, Nature, 365:566 (1993); Carlsson, et al., Nature, 380:207 (1996)). Other analog nucleic acids include those with positive backbones (Denpcy, et al., Proc. Natl. Acad. Sci. USA, 92:6097 (1995)); non-ionic backbones (U.S. Patent Nos. 5,386,023; 5,637,684; 5,602,240; 5,216,141; and 4,469,863; Kiedrowshi, et al., Angew. Chem. Intl. Ed. English, 30:423 (1991); Letsinger, et al., J. Am. Chem. Soc., 110:4470 (1988); Letsinger, et al., Nucleosides & Nucleotides, 13:1597 (1994); Chapters 2 and 3, ASC Symposium Series 580, "Carbohydrate Modifications in Antisense Research", Ed. Y.S. Sanghui and P. Dan Cook; Mesmaeker, et al., Bioorganic & Medicinal Chem. Lett., 4:395 (1994); Jeffs, et al., J. Biomolecular NMR, 34:17 (1994); Tetrahedron Lett., 37:743 (1996)) and non-ribose backbones, including those described in U.S. Patent Nos. 5,235,033 and 5,034,506, and Chapters 6 and 7, ASC Symposium Series 580, "Carbohydrate Modifications in Antisense Research", Ed. Y.S. Sanghui and P. Dan Cook. Nucleic acids containing one or more carbocyclic sugars are also included within the definition of nucleic acids (see Jenkins, et al., Chem. Soc. Rev., (1995) pp. 169-176). Several nucleic acid analogs are described in Rawls, C & E News, June 2, 1997, page 35. These modifications of the ribose-phosphate backbone may be done to facilitate the addition of additional moieties such as labels, or to increase the stability and half-life of such molecules in physiological environments; for example, PNA is particularly preferred. In addition, mixtures of naturally occurring nucleic acids and analogs can be made. Alternatively, mixtures of different nucleic acid analogs, and mixtures of naturally occurring nucleic acids and analogs may be made. The nucleic acids may be single stranded or double stranded, as specified, or contain portions of both double stranded or single stranded sequence. The nucleic acid may be DNA, both genomic and cDNA, RNA or a hybrid, where the nucleic acid contains any combination of deoxyribo- and ribo-nucleotides, and any combination of bases, including uracil, adenine, thymine, cytosine, guanine, inosine, xanthanine, hypoxanthanine, isocytosine, isoguanine, and base analogs such as nitropyrrole and nitroindole, etc.

As described above generally for proteins, nucleic acid bioactive agents may be naturally occuring nucleic acids, random nucleic acids, or "biased" random nucleic acids. For example, digests of procaryotic or eukaryotic genomes may be used as is outlined above for proteins.

In a preferred embodiment, as is more fully described below, the candidate agent comprises a fluorophore, that will exhibit a change in its fluorescent properties upon interaction (binding) with a target analyte.

The candidate agent is linked, preferably covalently, to the bead with a scissile linkage, such that the candidate agent can be cleaved from the bead prior to or during the assay as is more fully described below. By "scissile" or "cleavable" linkage herein is meant a linkage that is cleavable under assay conditions; that is, the candidate bioactive agent can be separated from the bead, resulting in a molecule that is essentially free to diffuse throughout the interior and exterior of the bead. In general, covalent linkages that are scissile are preferred, although non-covalent scissile linkages can also be used.

In a preferred embodiment, the scissile linkage is a photocleavable linkage, that is, a linkage that will be cleaved by exposure to light, particularly specific wavelengths of light. Thus, for example, photocleavable linkers such as ortho-nitrobenzyl groups, α-methylphenacyl ester, etc. may be used. See for example A Practical Guide to Combinatorial Chemistry, Eds. A. W, Czarnik and S.H. DeWitt, American Chemical Society, Washington D.C., 1997. A preferred photocleavable linker is shown below:

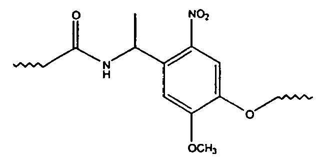

In a preferred embodiment, all the different candidate agents are attached to the beads using a single type of photocleavable linker, such that a single uniform application of light at the active wavelength will cause all of the agents to be released simultaneously. Alternatively, different photocleavable linkers may be used, for example to allow sequential monitoring of reactions, or "batch" analysis.

In a preferred embodiment, the scissile linkage may be an enzymatically cleavable linkage.
Depending on the composition of the candidate agent and the desired linkage, there are any number of suitable cleavage enzymes with known cleavage sites that can be used, including, but not limited to, hydrolases such as proteases, carbohydrases and lipases. For example, cleavable linkers may be used to attach the agents to the beads; for example, a protease may be used when the linker is a protein, a carbohydrase may be used when the linker is a carbohydrate, a lipase may be used when the linker is a lipid, etc.

As will be appreciated by those in the art, there are a wide variety of enzymatically cleavable sites that may be used to attach the agents to the beads. For example, sequences that are recognized and cleaved by a protease or cleaved after exposure to certain chemicals are considered cleavable linkers. For example, cleavable linkers include, but are not limited to, the prosequence of bovine chymosin, the prosequence of subtilisin, prosequences of retroviral proteases including human immunodeficiency virus protease and sequences recognized and cleaved by trypsin (EP 578472, Takasuga et al., J. Biochem. 112(5)652 (1992)) factor Xₐ (Gardella et al., J. Biol. Chem. 265(26):15854 (1990), WO 9006370), collagenase (J03280893, Tajima et al., J. Ferment. Bioeng. 72(5):362 (1991), WO 9006370), clostripain (EP 578472), subtilisin (including mutant H64A subtilisin, Forsberg et al., J. Protein Chem. 10(5):517 (1991), chymosin, yeast KEX2 protease (Bourbonnais et al., J. Bio. Chem. 263(30):15342 (1988), thrombin (Forsberg et al., supra; Abath et al., BioTechniques 10(2):178 (1991)), *Staphylococcus aureus* V8 protease or similar endoproteinase-Glu-C to cleave after Glu residues (EP 578472, Ishizaki et al., Appl. Microbiol. Biotechnol. 36(4):483 (1992)), cleavage by Nla proteainase of tobacco etch virus (Parks et al., Anal. Biochem. 216(2):413 (1994)), endoproteinase-Lys-C (U.S. Patent No. 4,414,332) and endoproteinase-Asp-N, Neisseria type 2 IgA protease (Pohlner et al., Bio/Technology 10(7):799-804 (1992)), soluble yeast endoproteinase yscF (EP 467839), chymotrypsin (Altman et al., Protein Eng. 4(5):593 (1991)), enteropeptidase (WO 9006370), lysostaphin, a polyglycine specific endoproteinase (EP 316748), and the like. See e.g. Marston, F.A.O. (1986) Biol. Chem. J. 240, 1-12.

Other types of labile linkages may be used, as will be appreciated by those in the art. For example, the scissile linkage may be an acid labile linkage, and other cleavable linkages. Particular amino acid sites that serve as chemical cleavage sites include, but are not limited to, methionine for cleavage by cyanogen bromide (Shen, PNAS USA 81:4627 (1984); Kempe et al., Gene 39:239 (1985); Kuliopulos et al., J. Am. Chem. Soc. 116:4599 (1994); Moks et al., Bio/Technology 5:379 (1987); Ray et al., Bio/Technology 11:64 (1993)), acid cleavage of an Asp-Pro bond (Wingender et al., J. Biol. Chem. 264(8):4367 (1989); Gram et al., Bio/Technology 12:1017 (1994)), and hydroxylamine cleavage at an Asn-Gly bond (Moks supra). Thus, suitable linkers include, but are not limited to, p-hydroxymethylbenzoic acid, 4-hydroxymethylphenylacetic acid, benzhydrylamino, allyl, hydroxyl-crontonyl-aminomethyl, 3-nitro-4-hydroxymethylbenzoic acid, p-nitrobenzyhydrylamine and 4-[4,4'-bis(methylsulfinyl)-2-oxy-benzhydrylamino]butyric acid.

In a preferred embodiment, each bead comprises a single type of bioactive agent, although a plurality of individual bioactive agents are preferably attached to each bead. Similarly, preferred embodiments utilize more than one microsphere containing a unique bioactive agent; that is, there is redundancy built into the system by the use of subpopulations of microspheres, each microsphere in the subpopulation containing the same bioactive agent.

In addition, as is more fully outlined below, the candidate agents may further comprise detectable labels that will alter their characteristics upon interaction (binding) with a target analyte.

As will be appreciated by those in the art, the bioactive agents may either be synthesized directly on the beads, or they may be made and then attached after synthesis.

In a preferred embodiment, the bioactive agents are synthesized directly on the beads. As is known in the art, many classes of chemical compounds are currently synthesized on solid supports, such as peptides, organic moieties, and nucleic acids. It is a relatively straightforward matter to adjust the current synthetic techniques to use porous beads.

In a preferred embodiment, the bioactive agents are synthesized first, and then covalently attached to the beads. As will be appreciated by those in the art, this will be done depending on the composition of the bioactive agents, the scissile linkage and the beads. The functionalization of solid support surfaces such as certain polymers with chemically reactive groups such as thiols, amines, carboxyls, etc. is generally known in the art. Accordingly, "blank" microspheres may be used that have surface chemistries that facilitate the attachment of the desired functionality by the user. Some examples of these surface chemistries for blank microspheres include, but are not limited to, amino groups including aliphatic and aromatic amines, carboxylic acids, aldehydes, amides, chloromethyl groups, hydrazide, hydroxyl groups, sulfonates and sulfates.

These functional groups can be used to add any number of different candidate agents to the beads, generally using known chemistries. For example, candidate agents containing carbohydrates may be attached to an amino-functionalized support; the aldehyde of the carbohydrate is made using standard techniques, and then the aldehyde is reacted with an amino group on the surface. In an alternative embodiment, a sulfhydryl linker may be used. There are a number of sulfhydryl reactive linkers known in the art such as SPDP, maleimides, α-haloacetyls, and pyridyl disulfides (see for example the 1994 Pierce Chemical Company catalog, technical section on cross-linkers, pages 155-200) which can be used to attach cysteine containing proteinaceous agents to the support. Alternatively, an amino group on the candidate agent may be used for attachment to an amino group on the surface. For example, a large number of stable bifunctional groups are well known in the art, including homobifunctional and heterobifunctional linkers (see Pierce Catalog and Handbook, pages 155-200). In an additional embodiment, carboxyl groups (either from the surface or from the candidate agent) may be derivatized using well known linkers (see the Pierce catalog). For example, carbodiimides activate carboxyl groups for attack by good nucleophiles such as amines (see Torchilin et al., Critical Rev. Therapeutic Drug Carrier Systems, 7(4):275-308 (1991)). Proteinaceous candidate agents may also be attached using other techniques known in the art, for example for the attachment of antibodies to polymers; see Slinkin et al., Bioconj. Chem. 2:342-348 (1991); Torchilin et al., supra; Trubetskoy et al., Bioconj. Chem. 3:323-327 (1992); King et al., Cancer Res. 54:6176-6185 (1994); and Wilbur et al., Bioconjugate Chem. 5:220-235 (1994)). It should be understood that the candidate agents may be attached in a variety of ways, including those listed above. What is important is that manner of attachment allows the incorporation of a scissile linkage and does not significantly alter the functionality of the candidate agent.

In some embodiments, there may be two species linked to the beads, using the techniques outlined above. Thus, for example, a bioactive agent being tested as an inhibitor may be synthesized on the beads, and a known enzyme substrate may be attached subsequently, using either the same cleavable linkage or a different one. Upon exposure to the cleavage agent, both molecules are released, allowing competitive assay analysis. Thus, some embodiments utilize beads comprising more than one assay component, all of which can be either synthesized on the bead, added to the bead after synthesis, or both. In addition, there may be one species linked to the bead and a second species is diffused in prior to the assay.

The present invention further provides a unique high-throughput screening advantage: the ability to vary the concentration of the agent in the assay from bead to bead, thus allowing concentration effects to be monitored simultaneously. This may be done in a number of ways, as will be appreciated by those in the art. As outlined above, this provides a number of significant advantages, including the ability to monitor kinetic parameters and thus avoid resynthesis of the agents and reassaying.

In a preferred embodiment, the number of "active sites" for chemical synthesis on and inside the beads is varied. That is, as is well known in the art, the number or density of synthetic sites on a surface such as a bead can be varied. This will allow the concentration of the candidate agents in an assay to vary from bead to bead. That is, since the amount of chemical synthesis will be determined by the number of active sites within the bead, the amount of agent synthesized can be varied. Since the assay "volume" is effectively the same from bead to bead, upon release of the agent from the bead prior to or during the assay, the effective concentration of the agent in the assay will be different for the different beads. By coding the beads according to the number of active sites on the bead, as is more fully outlined below, the effects of concentration differencs can be observed.

In a preferred embodiment, the number of active sites is not altered, but the functionality of the active sites is varied by adding capping agents and/or protecting agents. That is, a set number of active sites may be exposed to different concentrations of capping agents, resulting in different amounts of capping or inactivation of the sites. This may be done with protecting groups as well, where a standard synthetic protecting group is added to some active sites, followed by capping. Alternatively, mixtures of capping agent and protecting agents are made in different ratios, resulting in different amounts of available active groups for addition of the agents.

In a preferred embodiment, this concentration difference can be generated not by altering the number of active sites for synthesis, but by altering the composition of the cleavable linker such that the candidate agents are released at different rates. Thus for example, a first cleavable linker that results in fast cleavage is used for a first concentration of agent; a second cleavable linker that is slower is used for a second (lower) concentration of agent, etc.

In a preferred embodiment, this concentration difference can be generated by altering the amount of the cleavage agent introduced to the beads. For example, the same photocleavable linker may be used, but the amount of light, i.e. the number of photons, introduced to different beads is varied, resulting in different amounts of release. This may be done by using different "masking" techniques, as is well known in the art. This may provide the additional benefit of allowing the use of fewer codes, in that by using masks, spatial information about the location of different concentrations of agents can be acquired.

The microspheres further comprise an identifier moiety. As is more fully outlined below, the microspheres may be randomly associated with a substrate, such that each bead is localized at a discrete yet random site on the substrate. That is, the microspheres, each carrying different chemical functionalities are distributed on a substrate comprising a patterned surface of discrete sites that can bind the individual microspheres. The beads are generally put onto the substrate randomly, and thus several different methodologies can be used to "decode" the arrays, i.e. correlate the identity of a candidate agent with a location on the array. This is generally done using identifier moieties, which can utilize several different mechanisms to result in decoding of the random array. In one embodiment, the identifier moieties are unique optical signatures that are incorporated into the beads, for example fluorescent dyes, that can be used to optically identify the chemical functionality on any particular bead; other types of non-optical identifier moieties may also be used, as is described below. Alternatively, the identifier moiety may be an identifier binding ligand (IBL), to which a decoding binding ligand (DBL) will bind, allowing identification of the candidate agent bound to the bead. All of these methods allow the synthesis of the candidate agents (i.e. compounds such as nucleic acids and antibodies) to be divorced from their placement on an array, i.e. the candidate agents may be synthesized on the beads, and then the beads are randomly distributed on a patterned surface. Since the beads are first coded with an identifier moiety, this means that the array can later be "decoded", i.e. after the array is made, a correlation of the location of an individual site on the array with the bead or candidate agent at that particular site can be made. In addition, decoding may be accomplished by positional decoding, for example by either targeting the placement of beads (for example by using photoactivatible or photocleavable moieties to allow the selective addition of beads to particular locations), or by using either sub-bundles or selective loading of the sites, as are more fully outlined below. As is also discussed below, decoding may occur either prior to or after addition of a target analyte. The development of the subsequent decoding technology means that the beads may be randomly distributed on the array, a fast and inexpensive process as compared to either the in situ synthesis or spotting techniques of the prior art. These methods are generally outlined in WO 9840726.

In addition, the identifier moieties are not detached or removed from the beads for identification. That is, correlation of the identifier moiety and the location of the bead generally happens in situ, with the bead still on the array. This is in contrast to the "mass tags" types of systems described in U.S. patent No. 5,836,083; thus, the identifier moieties are not removable gas-chromatograph or mass-spectroscopy tags as are known in the art. However, in some embodiments, after decoding, it may be possible to remove the identifier moieties so that they do not interfere with any subsequent reaction.

Accordingly, in a preferred embodiment, the identifier moiety is an optical signature that can be used to identify the attached bioactive agent. That is, in this embodiment, each subpopulation of microspheres comprises a unique optical signature or optical tag that can be used to identify the unique bioactive agent of that subpopulation of microspheres; a bead comprising the unique optical signature may be distinguished from beads at other locations with different optical signatures. As is outlined herein, each bioactive agent will have an associated unique optical signature such that any microspheres comprising that bioactive agent will be identifiable on the basis of the signature. As is more fully outlined below, it is possible to reuse or duplicate optical signatures within an array, for example, when another level of identification is used, for example when beads of different sizes are used, or when the array is loaded sequentially with different batches of beads.

In a preferred embodiment, the optical signature is generally a mixture of reporter dyes, preferably fluorescent. By varying both the composition of the mixture (i.e. the ratio of one dye to another) and the concentration of the dye (leading to differences in signal intensity), matrices of unique tags may be generated. This may be done by covalently attaching the dyes to the surface of the beads, or alternatively, by entrapping the dye within the bead. The dyes may be chromophores or phosphors but are preferably fluorescent dyes, which due to their strong signals provide a good signal-to-noise ratio for decoding. Suitable dyes for use in the invention include, but are not limited to, fluorescent lanthanide complexes, including those of Europium and Terbium, fluorescein, rhodamine, tetramethylrhodamine, eosin, erythrosin, coumarin, methyl-coumarins, pyrene, Malacite green, stilbene, Lucifer Yellow, Cascade Blue™, Texas Red, and others described in the 6th Edition of Molecular Probes Handbook by Richard P. Haugland.

In a preferred embodiment, the encoding can be accomplished in a ratio of at least two dyes, although more encoding dimensions may be added in the size of the beads, for example. In addition, the labels are distinguishable from one another; thus two different labels may comprise different molecules (i.e. two different fluors) or, alternatively, one label at two different concentrations or intensity.

In a preferred embodiment, the dyes are covalently attached to the surface of the beads. This may be done as is generally outlined for the attachment of the bioactive agents, using functional groups on the surface of the beads. As will be appreciated by those in the art, these attachments are done to minimize the effect on the dye.

In a preferred embodiment, the dyes are non-covalently associated with the beads, generally by entrapping the dyes in the bead matrix or pores of the beads. Additionally, encoding in the ratios of the two or more dyes, rather than single dye concentrations, is preferred since it provides insensitivity to the intensity of light used to interrogate the reporter dye's signature and detector sensitivity.

In a preferred embodiment, the identifier moiety is an identifier binding ligand. By "identifier binding ligands" or "IBLs" herein is meant a compound that will specifically bind a corresponding decoder binding ligand (DBL) to facilitate the elucidation of the identity of the bioactive agent attached to the bead. That is, the IBL and the corresponding DBL form a binding partner pair. By "specifically bind" herein is meant that the IBL binds its DBL with specificity sufficient to differentiate between the corresponding DBL and other DBLs (that is, DBLs for other IBLs), or other components or contaminants of the system. The binding should be sufficient to remain bound under the conditions of the decoding step, including wash steps to remove non-specific binding. In some embodiments, for example when the IBLs and corresponding DBLs are proteins or nucleic acids, the dissociation constants of the IBL to its DBL will be less than about 10⁻⁴-10⁻⁶ M⁻¹, with less than about 10⁻⁵ to 10⁻⁹ M⁻¹ being preferred and less than about 10⁻⁷ -10⁻⁹ M⁻¹ being particularly preferred.

IBL-DBL binding pairs are known or can be readily found using known techniques. For example, when the IBL is a protein, the DBLs include proteins (particularly including antibodies or fragments thereof (FAbs, etc.)) or small molecules, or vice versa (the IBL is an antibody and the DBL is a protein). Metal ion- metal ion ligands or chelators pairs are also useful. Antigen-antibody pairs, enzymes and substrates or inhibitors, other protein-protein interacting pairs, receptor-ligands, complementary nucleic acids, and carbohydrates and their binding partners are also suitable binding pairs. Nucleic acid - nucleic acid binding proteins pairs are also useful. Similarly, as is generally described in U.S. Patents 5,270,163, 5,475,096, 5,567,588, 5,595,877, 5,637,459, 5,683,867,5,705,337, and related patents nucleic acid "aptamers" can be developed for binding to virtually any target; such a aptamer-target pair can be used as the IBL-DBL pair. Similarly, there is a wide body of literature relating to the development of binding pairs based on combinatorial chemistry methods.

As for the agent-target binding, a preferred embodiment utilizes IBLs that are molecules whose color or luminescence properties change in the presence of a selectively-binding DBL. For example, the IBL may be a fluorescent pH indicator whose emission intensity changes with pH. Similarly, the IBL may be a fluorescent ion indicator, whose emission properties change with ion concentration. Alternatively, the IBL is a molecule whose color or luminescence properties change in the presence of various solvents. For example, the IBL may be a fluorescent molecule such as an ethidium salt whose fluorescence intensity increases in hydrophobic environments. Similarly, the IBL may be a derivative of fluorescein whose color changes between aqueous and nonpolar solvents.

In one embodiment, the DBL may be attached to a bead, i.e. a "decoder bead", that may carry a label such as a fluorophore.

In a preferred embodiment, the IBL-DBL pair comprise substantially complementary single-stranded nucleic acids. In this embodiment, the binding ligands can be referred to as "identifier probes" and "decoder probes". Generally, the identifier and decoder probes range from about 4 basepairs in length to about 1000, with from about 6 to about 100 being preferred, and from about 8 to about 40 being particularly preferred. What is important is that the probes are long enough to be specific, i.e. to distinguish between different IBL-DBL pairs, yet short enough to allow both a) dissociation, if necessary, under suitable experimental conditions, and b) efficient hybridization.

In general, probes of the present invention are designed to be complementary to a target sequence (i.e. a DBL), such that hybridization of the target and the probes of the present invention occurs. This complementarity need not be perfect; there may be any number of base pair mismatches that will interfere with hybridization between the target sequence and the single stranded nucleic acid probes of the present invention. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under the selected reaction conditions. High stringency conditions are known in the art; see for example Maniatis et al., Molecular Cloning: A Laboratory Manual, 2d Edition, 1989, and Short Protocols in Molecular Biology, ed. Ausubel, et al.. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Acid Probes, "Overview of principles of hybridization and the strategy of nucleic acid assays" (1993). Generally, stringent conditions are selected to be about 5-10°C lower than the thermal melting point (Tm) for the specific sequence at a defined ionic strength pH. The Tm is the temperature (under defined ionic strength, pH and nucleic acid concentration) at which 50% of the probes complementary to the target hybridize to the target sequence at equilibrium (as the target sequences are present in excess, at Tm, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g. 10 to 50 nucleotides) and at least about 60°C for long probes (e.g. greater than 50 nucleotides). Stringent conditions may also be achieved with the addition of destabilizing agents such as formamide. In another embodiment, less stringent hybridization conditions are used; for example, moderate or low stringency conditions may be used, as are known in the art; see Maniatis and Ausubel, supra, and Tijssen, supra.

Alternatively, the identifier moiety ("IM") need not necessarily bind to DBLs. That is, rather than elucidate the structure of the bioactive agent directly, the composition of the IMs may serve as the identifier. Thus, for example, a specific combination of IMs can serve to code the bead; antibodies to the individual IMs can then be used for decoding.

In a preferred embodiment, each subpopulation of beads comprises a plurality of different IBLs. By using a plurality of different IBLs to encode each bioactive agent, the number of possible unique codes is substantially increased. That is, by using one unique IBL per bioactive agent, the size of the array will be the number of unique IBLs (assuming no "reuse" occurs, as outlined below). However, by using a plurality of different IBLs per bead, n, the size of the array can be increased to 2ⁿ, when the presence or absence of each IBL is used as the indicator. For example, the assignment of 10 IBLs per bead generates a 10 bit binary code, where each bit can be designated as "1" (IBL is present) or "0" (IBL is absent). A 10 bit binary code has 2¹⁰ possible variants. However, as is more fully discussed below, the size of the array may be further increased if another parameter is included such as concentration or intensity; thus for example, if two different concentrations of the IBL are used, then the array size increases as 3ⁿ. Thus, in this embodiment, each individual bioactive agent in the array is assigned a combination of IBLs, which can be added to the beads prior to the addition of the bioactive agent, after, or during the synthesis of the bioactive agent, i.e. simultaneous addition of IBLs and bioactive agent components.

Alternatively, when the bioactive agent is a polymer of different residues, i.e. when the bioactive agent is a protein or nucleic acid, the combination of different IBLs can be used to elucidate the sequence of the protein or nucleic acid.

Thus, for example, using two different IBLs (IBL1 and IBL2), the first position of a nucleic acid can be elucidated: for example, adenosine can be represented by the presence of both IBL1 and IBL2; thymidine can be represented by the presence of IBL1 but not IBL2, cytosine can be represented by the presence of IBL2 but not IBL1, and guanosine can be represented by the absence of both. The second position of the nucleic acid can be done in a similar manner using IBL3 and IBL4; thus, the presence of IBL1, IBL2, IBL3 and IBL4 gives a sequence of AA; IBL1, IBL2, and IBL3 shows the sequence AT; IBL1, IBL3 and IBL4 gives the sequence TA, etc. The third position utilizes IBL5 and IBL6, etc. In this way, the use of 20 different identifiers can yield a unique code for every possible 10-mer.

The system is similar for proteins but requires a larger number of different IBLs to identify each position, depending on the allowed diversity at each position. Thus for example, if every amino acid is allowed at every position, five different IBLs are required for each position. However, as outlined above, for example when using random peptides as the bioactive agents, there may be bias built into the system; not all amino acids may be present at all positions, and some positions may be preset; accordingly, it may be possible to utilize four different IBLs for each amino acid.

In this way, a sort of "bar code" for each sequence can be constructed; the presence or absence of each distinct IBL will allow the identification of each bioactive agent.

In addition, the use of different concentrations or densities of IBLs allows a "reuse" of sorts. If, for example, the bead comprising a first agent has a 1X concentration of IBL, and a second bead comprising a second agent has a 10X concentration of IBL, using sufficiently high (e.g. saturating) concentrations of the corresponding labelled DBL allows the user to distinguish between the two beads.

Accordingly, the beads of the invention comprise candidate bioactive agents and identifier moieties. In a preferred embodiment, the beads are distributed on the surface. In general, the methods of making the arrays and of decoding the arrays is done to maximzie the number of different candidate agents that can be uniquely encoded. The compositions of the invention may be made in a variety of ways. In general, the arrays are made by adding a solution or slurry comprising the beads to a surface containing the sites for attachment of the beads. This may be done in a variety of buffers, including aqueous and organic solvents, and mixtures. The solvent can evaporate, and excess beads removed. Alternatively, the surface comprising the sites, particularly wells, can be dipped into a dry mixture of the beads, and the excess "tapped" off.

In a preferred embodiment, when non-covalent methods are used to associate the beads to the array, a novel method of loading the beads onto the array is used. This method comprises exposing the array to a solution of particles and then applying energy, e.g. agitating or vibrating the mixture. This results in an array comprising more tightly associated particles, as the agitation is done with sufficient energy to cause weakly-associated beads to fall off (or out, in the case of wells). These sites are then available to bind a different bead. In this way, beads that exhibit a high affinity for the sites are selected. Arrays made in this way have two main advantages as compared to a more static loading: first of all, a higher percentage of the sites can be filled easily, and secondly, the arrays thus loaded show a substantial decrease in bead loss during assays. Thus, in a preferred embodiment, these methods are used to generate arrays that have at least about 50% of the sites filled, with at least about 75% being preferred, and at least about 90% being particularly preferred. Similarly, arrays generated in this manner preferably lose less than about 20% of the beads during an assay, with less than about 10% being preferred and less than about 5% being particularly preferred.

In this embodiment, the substrate comprising the surface with the discrete sites is immersed into a solution comprising the particles. The surface may comprise wells, as is described herein, or other types of sites on a patterned surface such that there is a differential affinity for the sites. This differential affinity results in a competitive process, such that particles that will associate more tightly are selected. Preferably, the entire surface to be "loaded" with beads is in fluid contact with the solution. This solution is generally a slurry ranging from about 10,000:1 beads:solution (vol:vol) to 1:1. Generally, the solution can comprise any number of reagents, including aqueous buffers, organic solvents, salts, other reagent components, etc. In addition, the solution preferably comprises an excess of beads; that is, there are more beads than sites on the array. Preferred embodiments utilize two-fold to billion-fold excess of beads.

The immersion can mimic the assay conditions; for example, if the array is to be "dipped" from above into a microtiter plate comprising samples, this configuration can be repeated for the loading, thus minimizing the beads that are likely to fall out due to gravity.

Once the surface has been immersed, the substrate, the solution, or both are subjected to a competitive process, whereby the particles with lower affinity can be disassociated from the substrate and replaced by particles exhibiting a higher affinity to the site. This competitive process is done by the introduction of energy, in the form of heat, sonication, stirring or mixing, vibrating or agitating the solution or substrate, or both.

A preferred embodiment utilizes agitation or vibration. In general, the amount of manipulation of the substrate is minimized to prevent damage to the array; thus, preferred embodiments utilize the agitation of the solution rather than the array, although either will work. As will be appreciated by those in the art, this agitation can take on any number of forms, with a preferred embodiment utilizing microtiter plates comprising bead solutions being agitated using microtiter plate shakers.

The agitation proceeds for a period of time sufficient to load the array to a desired fill. Depending on the size and concentration of the beads and the size of the array, this time may range from about 1 second to days, with from about 1 minute to about 24 hours being preferred.

It should be noted that not all sites of an array may comprise a bead; that is, there may be some sites of the substrate surface which are empty. In addition, there may be some sites that contain more than one bead, although this is generally not preferred.

In a preferred embodiment, the compositions of the invention are made in a composite array format.

In one embodiment, the beads are not distributed on a surface, but are used in solution, with reaction monitoring and decoding occurring using a FACS, for example. In this embodiment, the beads are preferably put into a diffusion-retarding solvent, for example an organic solvent such as hexane, such that the reaction components do not freely diffuse out of the bead. High viscosity aqueous solvents can also be used.

In some embodiments, for example when chemical attachment is done, it is possible to attach the beads in a non-random or ordered way. For example, using photoactivatible attachment linkers or photoactivatible adhesives or masks, selected sites on the array may be sequentially rendered suitable for attachment, such that defined populations of beads are laid down.

The arrays of the present invention are constructed such that information about the identity of the candidate agent is built into the array, such that the random deposition of the beads on the surface, for example in fiber wells, can be "decoded" to allow identification of the candidate agent at all positions. This may be done in a variety of ways, and either before, during or after the use of the array to detect target molecules.

Thus, after the array is made, it is "decoded" in order to identify the location of one or more of the bioactive agents, i.e. each subpopulation of beads, on the substrate surface.

In a preferred embodiment, a selective decoding system is used. In this case, only those microspheres exhibiting a change in the optical signal as a result of the binding of a target analyte are decoded. This is commonly done when the number of "hits", i.e. the number of sites to decode, is generally low. That is, the array is first scanned under experimental conditions in the absence of the target analytes. The sample containing the target analytes is added, and only those locations exhibiting a change in the optical signal are decoded. For example, the beads at either the positive or negative signal locations may be either selectively tagged or released from the array (for example through the use of photocleavable linkers generally different from those used to link the candidate agents to the beads), and subsequently sorted or enriched in a fluorescence-activated cell sorter (FACS). That is, either all the negative beads are released, and then the positive beads are either released or analyzed in situ, or alternatively all the positives are released and analyzed. Alternatively, the labels may comprise halogenated aromatic compounds, and detection of the label is done using for example gas chromatography, chemical tags, isotopic tags, or mass spectral tags.

As will be appreciated by those in the art, this may also be done in systems where the array is not decoded; i.e. there need not ever be a correlation of bead composition with location. In this embodiment, the beads are loaded on the array, and the assay is run. The "positives", i.e. those beads displaying a change in the optical signal as is more fully outlined below, are then "marked" to distinguish or separate them from the "negative" beads. This can be done in several ways, preferably using fiber optic arrays. In a preferred embodiment, each bead contains a fluorescent dye. After the assay and the identification of the "positives" or "active beads", light is shown down either only the positive fibers or only the negative fibers, generally in the presence of a light-activated reagent (typically dissolved oxygen). In the former case, all the active beads are photobleached. Thus, upon non-selective release of all the beads with subsequent sorting, for example using a fluorescence activated cell sorter (FACS) machine, the non-fluorescent active beads can be sorted from the fluorescent negative beads. Alternatively, when light is shown down the negative fibers, all the negatives are non-fluorescent and the the postives are fluorescent, and sorting can proceed. The characterization of the attached bioactive agent may be done directly, for example using mass spectroscopy.

Alternatively, the identification may occur through the use of identifier moieties that need not necessarily bind to DBLs. That is, rather than elucidate the structure of the bioactive agent directly, the composition of the IMs may serve as the identifier. Thus, for example, a specific combination of IMs can serve to code the bead, and be used to identify the agent on the bead upon release from the bead followed by subsequent analysis, for example using a gas chromatograph or mass spectroscope.

Alternatively, rather than having each bead contain a fluorescent dye, each bead comprises a non-fluorescent precursor to a fluorescent dye. For example, using photocleavable protecting groups (again, generally different from the photocleavable linkers attaching the candidate agents, although in some embodiments they may be the same), such as certain ortho-nitrobenzyl groups, on a fluorescent molecule, photoactivation of the fluorochrome can be done. After the assay, light is shown down again either the "positive" or the "negative" fibers, to distinquish these populations. The illuminated precursors are then chemically converted to a fluorescent dye. All the beads are then released from the array, with sorting, to form populations of fluorescent and non-fluorescent beads (either the positives and the negatives or vice versa).

In an alternate preferred embodiment, the sites of attachment of the beads (for example the wells) include a photopolymerizable reagent, or the photopolymerizable agent is added to the assembled array. After the test assay is run, light is shown down again either the "positive" or the "negative" fibers, to distinquish these populations. As a result of the irradiation, either all the positives or all the negatives are polymerized and trapped or bound to the sites, while the other population of beads can be released from the array.

In a preferred embodiment, the location of every bioactive agent is determined using decoder binding ligands (DBLs). As outlined above, DBLs are binding ligands that will either bind to identifier binding ligands, if present, or to the bioactive agents themselves, preferably when the bioactive agent is a nucleic acid or protein.

In a preferred embodiment, as outlined above, the DBL binds to the IBL.

In a preferred embodiment, the bioactive agents are single-stranded nucleic acids and the DBL is a substantially complementary single-stranded nucleic acid that binds (hybridizes) to the bioactive agent, termed a decoder probe herein. A decoder probe that is substantially complementary to each candidate probe is made and used to decode the array. In this embodiment, the candidate probes and the decoder probes should be of sufficient length (and the decoding step run under suitable conditions) to allow specificity; i.e. each candidate probe binds to its corresponding decoder probe with sufficient specificity to allow the distinction of each candidate probe.

In a preferred embodiment, the DBLs are either directly or indirectly labeled. By "labeled" herein is meant that a compound has at least one element, isotope or chemical compound attached to enable the detection of the compound. In general, labels fall into three classes: a) isotopic labels, which may be radioactive or heavy isotopes; b) magnetic, electrical, thermal; and c) colored or luminescent dyes; although labels include enzymes and particles such as magnetic particles as well. Preferred labels include luminescent labels, particularly fluorescent labels as outlined above for optical signatures. In a preferred embodiment, the DBL is directly labeled, that is, the DBL comprises a label. In an alternate embodiment, the DBL is indirectly labeled; that is, a labeling binding ligand (LBL) that will bind to the DBL is used. In this embodiment, the labeling binding ligand-DBL pair can be as described above for IBL-DBL pairs.

Accordingly, the identification of the location of the individual beads (or subpopulations of beads) is done using one or more decoding steps comprising a binding between the labeled DBL and either the IBL or the bioactive agent (i.e. a hybridization between the candidate probe and the decoder probe when the bioactive agent is a nucleic acid). After decoding, the DBLs can be removed and the array can be used; however, in some circumstances, for example when the DBL binds to an IBL and not to the bioactive agent, the removal of the DBL is not required (although it may be desirable in some circumstances). In addition, as outlined herein, decoding may be done either before the array is used in an assay, during the assay, or after the assay.

In one embodiment, a single decoding step is done. In this embodiment, each DBL is labeled with a unique label, such that the the number of unique tags is equal to or greater than the number of bioactive agents (although in some cases, "reuse" of the unique labels can be done, as described herein; similarly, minor variants of candidate probes can share the same decoder, if the variants are encoded in another dimension, i.e. in the bead size or label). For each bioactive agent or IBL, a DBL is made that will specifically bind to it and contains a unique tag, for example one or more fluorochromes. Thus, the identity of each DBL, both its composition (i.e. its sequence when it is a nucleic acid) and its label, is known. Then, by adding the DBLs to the array containing, the bioactive agents under conditions which allow the formation of complexes (termed hybridization complexes when the components are nucleic acids) between the DBLs and either the bioactive agents or the IBLs, the location of each DBL can be elucidated. This allows the identification of the location of each bioactive agent; the random array has been decoded. The DBLs can then be removed, if necessary, and the target sample applied.

In a preferred embodiment, the number of unique labels is less than the number of unique bioactive agents, and thus a sequential series of decoding steps are used. To facilitate the discussion, this embodiment is explained for nucleic acids, although other types of bioactive agents and DBLs are useful as well. In this embodiment, decoder probes are divided into n sets for decoding. The number of sets corresponds to the number of unique tags. Each decoder probe is labeled in n separate reactions with n distinct tags. All the decoder probes share the same n tags. The decoder probes are pooled so that each pool contains only one of the n tag versions of each decoder, and no two decoder probes have the same sequence of tags across all the pools. The number of pools required for this to be true is determined by the number of decoder probes and the n. Hybridization of each pool to the array generates a signal at every address. The sequential hybridization of each pool in turn will generate a unique, sequence-specific code for each candidate probe. This identifies the candidate probe at each address in the array. For example, if four tags are used, then 4 X n sequential hybridizations can ideally distinguish 4" sequences, although in some cases more steps may be required. After the hybridization of each pool, the hybrids are denatured and the decoder probes removed, so that the probes are rendered single-stranded for the next hybridization (although it is also possible to hybridize limiting amounts of target so that the available probe is not saturated. Sequential hybridizations can be carried out and analyzed by subtracting pre-existing signal from the previous hybridization).

An example is illustrative. Assuming an array of 16 probe nucleic acids (numbers 1-16), and four unique tags (four different fluors, for example; labels A-D). Decoder probes 1-16 are made that correspond to the probes on the beads. The first step is to label decoder probes 1-4 with tag A, decoder probes 5-8 with tag B, decoder probes 9-12 with tag C, and decoder probes 13-16 with tag D. The probes are mixed and the pool is contacted with the array containing the beads with the attached candidate probes. The location of each tag (and thus each decoder and candidate probe pair) is then determined. The first set of decoder probes is then removed. A second set is added, but this time, decoder probes 1, 5, 9 and 13 are labeled with tag A, decoder probes 2, 6, 10 and 14 are labeled with tag B, decoder probes 3, 7, 11 and 15 are labeled with tag C, and decoder probes 4, 8, 12 and 16 are labeled with tag D. Thus, those beads that contained tag A in both decoding steps contain candidate probe 1; tag A in the first decoding step and tag B in the second decoding step contain candidate probe 2; tag A in the first decoding step and tag C in the second step contain candidate probe 3; etc.

In one embodiment, the decoder probes are labeled in situ; that is, they need not be labeled prior to the decoding reaction. In this embodiment, the incoming decoder probe is shorter than the candidate probe, creating a 5' "overhang" on the decoding probe. The addition of labeled ddNTPs (each labeled with a unique tag) and a polymerase will allow the addition of the tags in a sequence specific manner, thus creating a sequence-specific pattern of signals. Similarly, other modifications can be done, including ligation, etc.

In addition, since the size of the array will be set by the number of unique decoding binding ligands, it is possible to "reuse" a set of unique DBLs to allow for a greater number of test sites. This may be done in several ways; for example, by using some subpopulations that comprise optical signatures. Similarly, the use of a positional coding scheme within an array; different sub-bundles may reuse the set of DBLs. Similarly, one embodiment utilizes bead size as a coding modality, thus allowing the reuse of the set of unique DBLs for each bead size. Alternatively, sequential partial loading of arrays with beads can also allow the reuse of DBLs. Furthermore, "code sharing" can occur as well.

In a preferred embodiment, the DBLs may be reused by having some subpopulations of beads comprise optical signatures. Thus, agents 1-100 are on non-labeled beads; agents 101-200 are on beads comprising a first label, etc. The unique combinations generated in this scheme allow the reuse of the DBL labels.

In a preferred embodiment, a spatial or positional coding system is done. In this embodiment, there are sub-bundles or subarrays (i.e. portions of the total array) that are utilized. By analogy with the telephone system, each subarray is an "area code", that can have the same tags (i.e. telephone numbers) of other subarrays, that are separated by virtue of the location of the subarray. Thus, for example, the same unique tags can be reused from bundle to bundle. Thus, the use of 50 unique tags in combination with 100 different subarrays can form an array of 5000 different bioactive agents. In this embodiment, it becomes important to be able to identify one bundle from another; in general, this is done either manually or through the use of marker beads, i.e. beads containing unique tags for each subarray.

In alternative embodiments, additional encoding parameters can be added, such as microsphere size. For example, the use of different size beads may also allow the reuse of sets of DBLs; that is, it is possible to use microspheres of different sizes to expand the encoding dimensions of the microspheres. Optical fiber arrays can be fabricated containing pixels with different fiber diameters or cross-sections; alternatively, two or more fiber optic bundles, each with different cross-sections of the individual fibers, can be added together to form a larger bundle; or, fiber optic bundles with fiber of the same size cross-sections can be used, but just with different sized beads. With different diameters, the largest wells can be filled with the largest microspheres and then moving onto progressively smaller microspheres in the smaller wells until all size wells are then filled. In this manner, the same dye ratio could be used to encode microspheres of different sizes thereby expanding the number of different oligonucleotide sequences or chemical functionalities present in the array. Although outlined for fiber optic substrates, this as well as the other methods outlined herein can be used with other substrates and with other attachment modalities as well.

In a preferred embodiment, the coding and decoding is accomplished by sequential loading of the microspheres into the array. As outlined above for spatial coding, in this embodiment, the optical signatures can be "reused". In this embodiment, the library of microspheres each comprising a different bioactive agent (or the subpopulations each comprise a different bioactive agent), is divided into a plurality of sublibraries; for example, depending on the size of the desired array and the number of unique tags, 10 sublibraries each comprising roughly 10% of the total library may be made, with each sublibrary comprising roughly the same unique tags. Then, the first sublibrary is added to the fiber optic bundle comprising the wells, and the location of each bioactive agent is determined, generally through the use of DBLs. The second sublibrary is then added, and the location of each bioactive agent is again determined. The signal in this case will comprise the signal from the "first" DBL and the "second" DBL; by comparing the two matrices the location of each bead in each sublibrary can be determined. Similarly, adding the third, fourth, etc. sublibraries sequentially will allow the array to be filled.

In a preferred embodiment, codes can be "shared" in several ways. In a first embodiment, a single code (i.e. IBL/DBL pair) can be assigned to two or more agents if the target analytes differ sufficiently in their binding strengths. For example, two nucleic acid probes used in an mRNA quantitation assay can share the same code if the ranges of their hybridization signal intensities do not overlap. This can occur, for example, when one of the target sequences is always present at a much higher concentration than the other. Alternatively, the two target sequences might always be present at a similar concentration, but differ in hybridization efficiency.

Alternatively, a single code can be assigned to multiple agents if the agents are functionally equivalent. For example, if a set of oligonucleotide probes are designed with the common purpose of detecting the presence of a particular gene, then the probes are functionally equivalent, even though they may differ in sequence. Similarly, if classes of analytes are desired, all probes for different members of a class such as kinases or G-protein coupled receptors could share a code. Similarly, an array of this type could be used to detect homologs of known genes. In this embodiment, each gene is represented by a heterologous set of probes, hybridizing to different regions of the gene (and therefore differing in sequence). The set of probes share a common code. If a homolog is present, it might hybridize to some but not all of the probes. The level of homology might be indicated by the fraction of probes hybridizing, as well as the average hybridization intensity. Similarly, multiple antibodies to the same protein could all share the same code.

It should be noted that while the decoding step is discussed prior to the assay step, decoding need not be done prior to the assay; decoding may occur before, during or after the assay is performed.

Once made, the compositions of the invention find use in a number of applications. In general, there are two major applications: a) high throughput assays of candidate agents to detect interactions with target analytes, and b) high throughput assays for the detection of the presence or absence of target analytes, for example nucleic acids and proteins.

In a preferred embodiment, assay methods are run for the detection of the interaction (binding) of candidate agents with target analytes. That is, there are a wide variety of assays which are traditionally run to detect novel interactions; for example, chemical libraries are screened to evalute their binding and/or bioeffect on any number of target analytes. Accordingly, the methods provide for the detection of binding of candidate agents to target analytes. By "binding" or "interaction" herein is meant a permanent or transitory physical interaction of the candidate agent and the target analyte.

By "target analyte" or "analyte" or grammatical equivalents herein is meant any atom, molecule, ion, molecular ion, compound or particle to be either detected or evaluated for binding partners. As will be appreciated by those in the art, a large number of analytes may be used in the present invention; basically, any target analyte can be used which binds a bioactive agent or for which a binding partner (i.e. drug candidate) is sought.

Suitable analytes include organic and inorganic molecules, including biomolecules. When detection of a target analyte is done, suitable target analytes include, but are not limited to, an environmental pollutant (including pesticides, insecticides, toxins, etc.); a chemical (including solvents, polymers, organic materials, etc.); therapeutic molecules (including therapeutic and abused drugs, antibiotics, etc.); biomolecules (including hormones, cytokines, proteins including enzymes, nucleic acids, lipids, carbohydrates, cellular membrane antigens and receptors (neural, hormonal, nutrient, and cell surface receptors) or their ligands, etc); whole cells (including procaryotic (such as pathogenic bacteria) and eukaryotic cells, including mammalian tumor cells); viruses (including retroviruses, herpesviruses, adenoviruses, lentiviruses, etc.); and spores; etc. Particularly preferred analytes are nucleic acids and proteins.

In a preferred embodiment, the target analyte is a protein. As will be appreciated by those in the art, there are a large number of possible proteinaceous target analytes that may be detected or evaluated for binding partners using the present invention. Suitable protein target analytes include, but are not limited to, (1) immunoglobulins; (2) enzymes (and other proteins); (3) hormones and cytokines (many of which serve as ligands for cellular receptors); and (4) other proteins.

In a preferred embodiment, the target analyte is a nucleic acid. These assays find use in a wide variety of applications, as is more fully outlined below.

In general, assays that are used to probe for novel interactions can be run in a variety of ways, as will be appreciated by those in the art. In general, since the assays are run within beads, assays requiring wash steps are not preferred. Accordingly, there are four major ways these assays may be accomplished: a) the candidate agent comprises a detectable moiety such as a fluorochrome that changes its detectable properties upon binding to the target analyte; b) the target analyte comprises the detectable moiety that alters upon binding to the candidate agent; c) both the target analyte and the candidate agent comprise detectable moieties that alter their properties upon binding; or d) one of the assay components comprises a detectable moiety that alters upon the binding of the agent to the target.

In a preferred embodiment, the candidate agent comprises a detectable moiety that changes its properties or characteristics upon binding to the target analyte. For example, as is well known in the art, many fluorochromes alter their fluorescent properties as a result of the relative hydrophobicity of their environment. Thus, for example, some fluorochromes frequently exhibit low fluorescence in aqueous environments, such as free in aqueous solution. However, upon sequestration into a hydrophobic environment, for example a binding pocket of a target analyte, the fluor increases its signal.

In a preferred embodiment, the target analyte comprises a detectable moiety that changes its properties or characteristics upon binding to the candidate agent. Thus, for example, as outlined above, the binding pocket of an enzyme may comprise a solvent accessible fluor that alters its fluorescent properties upon binding of a candidate agent.

In a preferred embodiment, both the target and the candidate agent comprise detectable moieties that alter their properties upon binding. For example, fluorescence resonance energy transfer (FRET) systems are well known. In a preferred embodiment, the system comprises detectable molecules formed of two fluorescent proteins, i.e., blue and green fluorescent protein (BFP and GFP), and other similar molecules. As is known in the art, constructs of GFP and BFP that hold these two proteins in close proximity allow fluorescence resonance energy transfer (FRET). That is, the excitation spectra of the GFP overlaps the emission spectra of the BFP. Accordingly, exciting the BFP results in GFP emission. If the candidate agent and target each comprise one of these labels, the interaction of these two form a detectable "FRET complex"; if the two do not interact, there is no GFP emission at the BFP excitation wavelength. The system is further described in Xu et al., Nucleic Acid Res. 26(8):2034 (1998); and Miyawaki et al., Nature 388(6645):882-887 (1997).

In a preferred embodiment, it is a component of the assay system that comprises a detectable moiety that alters as a result of the interaction of the target and candidate agent. For example, intercalators are molecules, many of which are fluorescent, that preferentially insert into double stranded nucleic acid. Upon binding, generally as a result of moving from a hydrophilic to a relatively hydrophobic environment, the fluorescence of the intercalator increases.

Similarly, in a preferred embodiment, the assay is directed to screening for enzymatic inhibitors, and the assay includes a substrate that becomes detectable as the enzyme acts upon it. That is, thereare a wide variety of chromogenic and fluorogenic enzymatic substrates known that can be added to the assay system. If the candidate agent is an effective inhibitor, a loss of signal is seen. Suitable enzymes for screening for inhibitors include, but are not limited to, viral, bacterial and parasitic enzymes and therapeutically relevant enzymes including, but not limited to, the cathepsins, caspases, other proteases, etc.

In a preferred embodiment, the compositions are used to probe a sample solution for the presence or absence of a target analyte, including the quantification of the amount of target analyte present. Thus, for example, a preferred embodiment utilizes nucleic acid probes as the candidate agents, to screen samples for the presence or absence of certain target sequences.

Thus, in a preferred embodiment, the probes are used in genetic diagnosis. For example, probes can be made using the techniques disclosed herein to detect target sequences such as the gene for nonpolyposis colon cancer, the BRCA1 breast cancer gene, P53, which is a gene associated with a variety of cancers, the Apo E4 gene that indicates a greater risk of Alzheimer's disease, allowing for easy presymptomatic screening of patients, mutations in the cystic fibrosis gene, cytochrome p450s or any of the others well known in the art.

In an additional embodiment, viral and bacterial detection is done using the complexes of the invention. In this embodiment, probes are designed to detect target sequences from a variety of bacteria and viruses. For example, current blood-screening techniques rely on the detection of anti-HIV antibodies. The methods disclosed herein allow for direct screening of clinical samples to detect HIV nucleic acid sequences, particularly highly conserved HIV sequences. In addition, this allows direct monitoring of circulating virus within a patient as an improved method of assessing the efficacy of anti-viral therapies. Similarly, viruses associated with leukemia, HTLV-I and HTLV-II, may be detected in this way. Bacterial infections such as tuberculosis, chlamydia and other sexually transmitted diseases, may also be detected.

In a preferred embodiment, the nucleic acids of the invention find use as probes for toxic bacteria in the screening of water and food samples. For example, samples may be treated to lyse the bacteria to release its nucleic acid, and then probes designed to recognize bacterial strains, including, but not limited to, such pathogenic strains as, Salmonella, Campylobacter, Vibrio cholerae, Leishmania, enterotoxic strains of E. coli, and Legionnaire's disease bacteria. Similarly, bioremediation strategies may be evaluated using the compositions of the invention.

In a further embodiment, the probes are used for forensic "DNA fingerprinting" to match crime-scene DNA against samples taken from victims and suspects.

In an additional embodiment, the probes in an array are used for sequencing by hybridization.

The present invention also finds use as a methodology for the detection of mutations or mismatches in target nucleic acid sequences. For example, recent focus has been on the analysis of the relationship between genetic variation and phenotype by making use of polymorphic DNA markers. Previous work utilized short tandem repeats (STRs) as polymorphic positional markers; however, recent focus is on the use of single nucleotide polymorphisms (SNPs), which occur at an average frequency of more than 1 per kilobase in human genomic DNA. Some SNPs, particularly those in and around coding sequences, are likely to be the direct cause of therapeutically relevant phenotypic variants. There are a number of well known polymorphisms that cause clinically important phenotypes; for example, the apoE2/3/4 variants are associated with different relative risk of Alzheimer's and other diseases (see Cordor et al., Science 261 (1993). Multiplex PCR amplification of SNP loci with subsequent hybridization to oligonucleotide arrays has been shown to be an accurate and reliable method of simultaneously genotyping at least hundreds of SNPs; see Wang et al., Science, 280:1077 (1998); see also Schafer et al., Nature Biotechnology 16:33-39 (1998). The compositions of the present invention may easily be substituted for the arrays of the prior art.

In a preferred embodiment, two-color competitive hybridization assays are run. These assays can be based on traditional sandwich assays. The beads contain a capture sequence located on one side (upstream or downstream) of the SNP, to capture the target sequence. Two SNP allele-specific probes, each labeled with a different fluorophor, are hybridized to the target sequence. The genotype can be obtained from a ratio of the two signals, with the correct sequence generally exhibiting better binding. This has an advantage in that the target sequence itself need not be labeled. In addition, since the probes are competing, this means that the conditions for binding need not be optimized. Under conditions where a mismatched probe would be stably bound, a matched probe can still displace it. Therefore the competitive assay can provide better discrimination under those conditions. Because many assays are carried out in parallel, conditions cannot be optimzed for every probe simultaneously. Therefore, a competitive assay system can be used to help compensate for non-optimal conditions for mismatch discrimination.

In a preferred embodiment, dideoxynucleotide chain-termination sequencing is done using the compositions of the invention. In this embodiment, a DNA polymerase is used to extend a primer using fluorescently labeled ddNTPs. The 3' end of the primer is located adjacent to the SNP site. In this way, the single base extension is complementary to the sequence at the SNP site. By using four different fluorophors, one for each base, the sequence of the SNP can be deduced by comparing the four base-specific signals. This may be done in several ways. In a first embodiment, the capture probe can be extended; in this approach, the probe must either be synthesized 5'-3' on the bead, or attached at the 5' end, to provide a free 3' end for polymerase extension. Alternatively, a sandwich type assay can be used; in this embodiment, the target is captured on the bead by a probe, then a primer is annealed and extended. Again, in the latter case, the target sequence need not be labeled.

In addition, since sandwich assays require two specific interactions, this provides increased stringency which is particularly helpful for the analysis of complex samples.

In addition, when the target analyte and the DBL both bind to the agent, it is also possible to do detection of non-labelled target analytes via competition of decoding.

In a preferred embodiment, the compositions of the invention are used to screen bioactive agents to find an agent that will bind, and preferably modify the function of, a target molecule. As above, a wide variety of different assay formats may be run, as will be appreciated by those in the art.

In a preferred embodiment, the binding of the bioactive agent and the target analyte is specific; that is, the bioactive agent specifically binds to the target analyte. By "specifically bind" herein is meant that the agent binds the analyte, with specificity sufficient to differentiate between the analyte and other components or contaminants of the test sample. However, as will be appreciated by those in the art, it will be possible to detect analytes using binding which is not highly specific; for example, the systems may use different binding ligands, for example an array of different ligands, and detection of any particular analyte is via its "signature" of binding to a panel of binding ligands, similar to the manner in which "electronic noses" work. This finds particular utility in the detection of chemical analytes. The binding should be sufficient to remain bound under the conditions of the assay, including wash steps to remove non-specific binding, although in some embodiments, wash steps are not desired; i.e. for detecting low affinity binding partners. In some embodiments, for example in the detection of certain biomolecules, the dissociation constants of the analyte to the binding ligand will be less than about 10⁻⁴-10⁻⁶ M⁻¹, with less than about 10⁻⁵ to 10⁻⁹ M⁻¹ being preferred and less than about 10⁻⁷ -10⁻⁹ M⁻¹ being particularly preferred.

Generally, a sample containing a target analyte (whether for detection of the target analyte or screening for binding partners of the target analyte) is added to the array, under conditions suitable for binding of the target analyte to at least one of the bioactive agents, i.e. generally physiological conditions. The sample or solution can additionally comprise a variety of other reagents. These include reagents like buffers, co-factors, enzyme substrates, intercalators, salts, neutral proteins, e.g. albumin, detergents, etc which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used.

Either before, during or after the introduction of the sample to the microspheres the scissile linkages may be cleaved, releasing the candidate agents into the bead.

An interaction between the candidate agent and the target analyte is then detected. As outlined herein, this can be done in a variety of ways, depending on whether screening assays or detection assays are done, although both types of assays rely on changes in detectable signals, generally optical signals, as a result of the interaction of the candidate agent and the target analyte.

When the assay is directed to the detection of the presence or absence of a target analyte, generally through the use of a change in an optical signal. This change can occur via many different mechanisms. A few examples include the binding of a dye-tagged analyte to the bead, the production of a dye species on or near the beads, the destruction of an existing dye species, a change in the optical signature upon analyte interaction with dye on bead, or any other optical interrogatable event.

In a preferred embodiment, the change in optical signal occurs as a result of the binding of a target analyte that is labeled, either directly or indirectly, with a detectable label, preferably an optical label such as a fluorochrome. Thus, for example, when a proteinaceous target analyte is used, it may be either directly labeled with a fluor, or indirectly, for example through the use of a labeled antibody. Similarly, nucleic acids are easily labeled with fluorochromes, for example during PCR amplification as is known in the art. Alternatively, upon binding of the target sequences, a hybridization indicator may be used as the label. Hybridization indicators preferentially associate with double stranded nucleic acid, usually reversibly. Hybridization indicators include intercalators and minor and/or major groove binding moieties. In a preferred embodiment, intercalators may be used; since intercalation generally only occurs in the presence of double stranded nucleic acid, only in the presence of target hybridization will the label light up. Thus, upon binding of the target analyte to a bioactive agent, there is a new optical signal generated at that site, which then may be detected.

Alternatively, in some cases, as discussed above, the target analyte such as an enzyme generates a species that is either directly or indirectly optical detectable.

Furthermore, in some embodiments, a change in the optical signature may be the basis of the optical signal. For example, the interaction of some chemical target analytes with some fluorescent dyes on the beads may alter the optical signature, thus generating a different optical signal.

As will be appreciated by those in the art, in some embodiments, the presence or absence of the target analyte may be done using changes in other optical or non-optical signals, including, but not limited to, surface enhanced Raman spectroscopy, surface plasmon resonance, radioactivity, etc.

When screening assays are done, detection proceeds via a change in a detectable signal as outlined above, generally using at least one of the four outlined mechanisms. The detectable signal is usually an optical signal, and preferably a fluorescent signal.

## Claims

1. An array composition comprising:
a) a substrate with a surface comprising discrete sites; and
b) a population of porous microspheres comprising at least a first and a second subpopulation, wherein each subpopulation comprises:
i) a candidate bioactive agent; and
ii) an identifier moiety;
wherein said microspheres are distributed on said surface, and **characterized in that** said microspheres are porous, said microspheres are associated with or attached to the discrete sites on the surface of the substrate,and said candidate bioactive agents are linked to said microspheres via a scissile linkage.

2. An array composition according to claim 1 wherein said identifier moiety is an identifier binding ligand that will bind a decoder binding ligand such that the identification of the candidate bioactive agent can be elucidated.

3. An array composition according to claim 2 wherein said identifier binding ligand is a nucleic acid probe.

4. An array composition according to claim 2 wherein said identifier binding ligand is a protein.

5. An array composition according to claim 1 wherein said identifier moiety is an optical signature.

6. An array composition according to claim 5 wherein said optical signature comprises a fluorescent dye.

7. An array composition according to claim 6 wherein said optical signature comprises two or more fluorescent dyes.

8. An array composition according to claim 1 wherein at least two subpopulations have different amounts of the same candidate agent.

9. An array composition comprising:
a) a substrate with a surface comprising discrete sites;
b) a population of porous microspheres comprising at least a first and a second subpopulation,
wherein said microspheres are distributed on said surface,
**characterized in that** said microspheres are associated with or attached to the discrete sites on the surface of the substrate and that said first and said second subpopulation each comprise the same candidate bioactive agent linked to said microspheres via a scissile linkage, wherein the amount of candidate bioactive agent on said first subpopulation is different from the amount of candidate bioactive agent on said second subpopulation.

10. An array composition according to claim 1 or 9 wherein said scissile linkage is a photocleavable linkage.

11. An array composition according to claim 1 or 9 wherein said scissile linkage is an enzyme cleavable linkage.

12. An assay method for detecting the binding of a candidate bioactive agent to a target analyte comprising:
a) adding a solution comprising at least one target analyte to a plurality of porous microspheres, wherein said microspheres are associated with or attached to the discrete sites on a surface of a substrate or are present in a diffusion-retarding solvent, and wherein said microspheres comprise at least a first and a second subpopulation, wherein each subpopulation comprises:
i) a candidate bioactive agent linked to said porous microspheres via a scissile linkage; and
ii) an identifier moiety;
b) cleaving said scissile linkage;
c) detecting the binding of at least one candidate bioactive agent to said target analyte.

13. A method according to claim 12 wherein said candidate bioactive agent comprises a fluorochrome that exhibits a change in fluorescence as a result of binding to said target analyte.

14. A method according to claim 12 wherein said target analyte comprises a fluorochrome that exhibits a change in fluorescence as a result of binding to said candidate bioactive agent.

15. A method according to claim 12 wherein said solution comprises a fluorochrome that exhibits a change in fluorescence as a result of the binding of said candidate bioactive agent to said target analyte.

16. A method according to claim 12 wherein said scissile linkage is a photocleavable linkage.

17. A method according to claim 12 wherein said scissile linkage is an enzymatically cleavable linkage.

18. A method according to claim 12 wherein said porous microspheres are distributed on a surface at discrete sites.

19. A method according to claim 13 wherein said identifier moiety is an identifier binding ligand that will bind a decoder binding ligand such that the identification of the candidate bioactive agent can be elucidated.

20. A method according to claim 19 wherein said identifier binding ligand is a nucleic acid probe.

21. A method according to claim 19 wherein said identifier binding ligand is a protein.

22. A method according to claim 19 wherein said identifier moiety is an optical signature.

23. A method according to claim 22 where said optical signature comprises a fluorescent dye.

24. A method according to claim 22 wherein said optical signature comprises two or more fluorescent dyes.

25. A method according to claim 12 wherein at least two subpopulations have different amounts of the same candidate bioactive agent.

26. A method according to claim 12 wherein said target analyte is a protein.

27. A method according to claim 26 wherein said protein is enzyme.

## Patentansprüche

1. Array-Zusammensetzung, bestehend aus:
a) einem Substrat mit einer Oberfläche, bestehend aus diskreten Stellen und
b) einer Population aus porösen Mikrosphären bestehend aus mindestens einer ersten und einer zweiter Subpopulation, wobei jede Subpopulation besteht aus:
i) einem bioaktiven Wirkstoffkandidat und
ii) einer identifizierenden Gruppe;
wobei besagte Mikrosphären auf besagter Oberflache verteilt sind und **dadurch gekennzeichnet, dass** besagte Mikrosphären porös sind, besagte Mikrosphären mit den diskreten Stellen auf der Oberfläche des Substrats assoziiert oder daran angebunden sind und besagte bioaktive Wirkstoffkandidaten mit besagten Mikrosphären via einer leicht spaltbaren Verbindung verbunden sind.

2. Array-Zusammensetzung nach Anspruch 1, wobei besagte identifizierende Gruppe ein identifizierender Bindungsligand ist, der einen Decoder-Bindungsliganden derart bindet, dass die Identifikation des bioaktiven Wirkstoffkandidaten verdeutlicht werden kann.

3. Array-Zusammensetzung nach Anspruch 2, wobei besagter identifizierender Bindungsligand eine Nukleinsäuresonde ist.

4. Array-Zusammensetzung nach Anspruch 2, wobei besagter identifizierender Bindungsligand ein Protein ist.

5. Array-Zusammensetzung nach Anspruch 1, wobei besagte identifizierende Gruppe eine optische Unterschrift ist.

6. Array-Zusammensetzung nach Anspruch 5, wobei besagte optische Unterschrift aus einem fluoreszierenden Farbstoff besteht.

7. Array-Zusammensetzung nach Anspruch 6, wobei besagte optische Unterschrift aus zwei oder mehreren fluoreszierenden Farbstoffen besteht.

8. Array-Zusammensetzung nach Anspruch 1, wobei mindestens zwei Subpopulationen verschiedene Mengen desselben Wirkstoffkandidaten aufweisen.

9. Array-Zusammensetzung bestehend aus:
a) einem Substrat mit einer Oberfläche bestehend aus diskreten Stellen;
b) einer Population aus porösen Mikrosphären, bestehend aus mindestens einer ersten und einer zweiten Subpopulation,
wobei besagte Mikrosphären auf besagter Oberfläche verteilt sind,
**dadurch gekennzeichnet, dass** besagte Mikrosphären mit den diskreten Stellen auf der Oberfläche des Substrats assoziiert oder daran angebunden sind und dass besagte erste und besagte zweite Subpopulation jede aus demselben bioaktiven Wirkstoffkandidat bestehen, der mit besagten Mikrosphären via einer leicht spaltbaren Verbindung verbunden ist, wobei die Menge des bioaktiven Wirkstoffkandidaten auf besagter erster Subpopulation von der Menge des bioaktiven Wirkstoffkandidaten auf besagter zweiter Subpopulation abweicht.

10. Array-Zusammensetzung nach Anspruch 1 oder 9, wobei besagte leicht spaltbare Verbindung eine durch Licht spaltbare Verbindung ist.

11. Array-Zusammensetzung nach Anspruch 1 oder 9, wobei besagte leicht spaltbare Verbindung eine durch Enzyme spaltbare Verbindung ist.

12. Analyseverfahren zur Feststellung der Bindung eines bioaktiven Wirkstoffkandidaten an ein Target-Analyt, bestehend aus:
a) Hinzufügen einer Lösung bestehend aus mindestens einem Target-Analyt zu einer Pluralität aus porösen Mikrosphären, wobei besagte Mikrosphären mit den diskreten Stellen auf der Oberfläche eines Substrats assoziiert oder daran angebunden sind oder in einer diffusionshemmenden Lösung vorhanden sind und wobei besagte Mikrosphären aus mindestens einer ersten und einer zweiten Subpopulation bestehen, wobei jede Subpopulation besteht aus:
i) einem bioaktiven Wirkstoffkandidat, der mit besagten porösen Mikrosphären über eine leicht spaltbare Verbindung verbunden ist und
ii) einer identifizierenden Gruppe;
b) Abspalten besagter leicht spaltbaren Verbindung;
c) Feststellen der Bindung von mindestens einem bioaktiven Wirkstoffkandidaten an besagtes Target-Analyt.

13. Verfahren nach Anspruch 12, wobei besagter bioaktive Wirkstoffkandidat aus einem Fluorochrom besteht, das aufgrund der Bindung an besagtes Target-Analyt eine Veränderung in der Fluoreszenz aufweist.

14. Verfahren nach Anspruch 12, wobei besagtes Target-Analy aus einem Fluorochrom besteht, das aufgrund der Bindung an besagten bioaktiven Wirkstoffkandidaten eine Veränderung in der Fluoreszenz aufweist.

15. Verfahren nach Anspruch 12, wobei besagte Lösung aus einem Fluorochrom besteht, das aufgrund der Bindung des besagten bioaktiven Wirkstoffkandidaten an besagtes Target-Analyt eine Veränderung in der Fluoreszenz aufweist.

16. Verfahren nach Anspruch 12, wobei besagte leicht spaltbare Verbindung eine durch Licht spaltbare Verbindung ist.

17. Verfahren nach Anspruch 12, wobei besagte leicht spaltbare Verbindung eine durch Enzyme spaltbare Verbindung ist.

18. Verfahren nach Anspruch 12, wobei besagte poröse Mikrosphären auf einer Oberfläche an diskreten Stellen verteilt sind.

19. Verfahren nach Anspruch 13, wobei besagte identifizierende Gruppe ein identifizierender Bindungsligand ist, der einen Dekoder-Bindungsliganden derart bindet, dass die Identifizierung des bioaktiven Wirkstoffkandidaten verdeutlicht werden kann.

20. Verfahren nach Anspruch 19, wobei besagter identifizierender Bindungsligand eine Nukleinsäuresonde ist.

21. Verfahren nach Anspruch 19, wobei besagter identifizierender Bindungsligand ein Protein ist.

22. Verfahren nach Anspruch 19, wobei besagte identifizierende Gruppe eine optische Unterschrift ist.

23. Verfahren nach Anspruch 22, wobei besagte optische Unterschrift aus einem fluoreszierenden Farbstoff besteht.

24. Verfahren nach Anspruch 22, wobei besagte optische Unterschrift aus zwei oder mehreren fluoreszierenden Farbstoffen besteht.

25. Verfahren nach Anspruch 12, wobei mindestens zwei Subpopulationen verschiedene Mengen desselben bioaktiven Wirkstoffkandidaten aufweisen.

26. Verfahren nach Anspruch 12, wobei besagtes Target-Analyt ein Protein ist.

27. Verfahren nach Anspruch 26, wobei besagtes Protein ein Enzym ist.

## Revendications

1. Composition de réseau comprenant :
a) un substrat ayant une surface comprenant des sites discrets ; et
b) une population de microsphères poreuses comprenant au moins une première sous-population et une deuxième sous-population, chaque sous-population comprenant :
i) un agent bioactif potentiel; et
ii) une entité identificatrice;
lesdites microsphères étant distribuées sur ladite surface, et **caractérisée en ce que** lesdites microsphères sont poreuses, lesdites microsphères étant associées aux sites discrets sur la surface du substrat, ou rattachées à ceux-ci, et lesdits agents bioactifs potentiels étant liés auxdites microsphères par le biais d'une liaison scissile.

2. Composition de réseau selon la revendication 1, dans laquelle ladite entité identificatrice est un ligand de liaison à l'identificateur qui se lie avec un ligand de liaison à un décodeur, de sorte que l'agent bioactif potentiel soit identifié.

3. Composition de réseau selon la revendication 2, dans laquelle ledit ligand de liaison à l'identificateur est une sonde d'acide nucléique.

4. Composition de réseau selon la revendication 2, dans laquelle ledit ligand de liaison à l'identificateur est une protéine.

5. Composition de réseau selon la revendication 1, dans laquelle ladite entité identificatrice est une signature optique.

6. Composition de réseau selon la revendication 5, dans laquelle ladite signature optique comprend un colorant fluorescent.

7. Composition de réseau selon la revendication 6, dans laquelle ladite signature optique comprend deux colorants fluorescents ou plus.

8. Composition de réseau selon la revendication 1, dans laquelle au moins deux sous-populations contiennent des quantités différentes du même agent potentiel.

9. Composition de réseau comprenant :
a) un substrat ayant une surface comprenant des sites discrets;
b) une population de microsphères poreuses comprenant au moins une première sous-population et une deuxième sous-population,
dans laquelle lesdites microsphères sont distribuées sur lesdites surfaces,
**caractérisée en ce que** lesdites microsphères sont associées aux sites discrets sur la surface du substrat, ou rattachées à ceux-ci, et que lesdites première sous-population et deuxième sous-population comprennent chacune le même agent bioactif potentiel lié auxdites microsphères par le biais d'une liaison scissile, dans laquelle la quantité dudit agent bioactif potentiel sur ladite première sous-population est différente de la quantité d'agent bioactif potentiel sur ladite deuxième population.

10. Composition de réseau selon la revendication 1 ou 9, dans laquelle ladite liaison scissile est une liaison photoclivable.

11. Composition de réseau selon la revendication 1 ou 9, dans laquelle ladite liaison scissile est une liaison clivable par une enzyme.

12. Procédé de dosage pour la détection de la liaison d'un agent bioactif potentiel à une substance à analyser ciblée comprenant :
a) l'ajout d'une solution comprenant au moins une substance à analyser ciblée à une multitude de microsphères poreuses, lesdites microsphères étant associées aux sites discrets sur la surface du substrat, ou rattachées à ceux-ci, ou sont présentes dans un solvant retardateur de diffusion, et lesdites microsphères comprenant au moins une première sous-population et une deuxième sous-population, chaque sous-population comprenant :
i) un agent bioactif potentiel lié auxdites microsphères poreuses par le biais d'une liaison scissile; et
ii) une entité identificatrice;
b) le clivage de ladite liaison scissile;
c) la détection de la liaison d'au moins un agent bioactif potentiel à ladite substance à analyser ciblée.

13. Procédé selon la revendication 12, dans lequel ledit agent bioactif potentiel comprend un fluorochrome qui présente un changement de fluorescence suite à la liaison avec ladite substance à analyser ciblée.

14. Procédé selon la revendication 12, dans lequel ladite substance à analyser ciblée comprend un fluorochrome qui présente un changement de fluorescence suite à la liaison avec ledit agent bioactif candidat.

15. Procédé selon la revendication 12, dans lequel ladite solution comprend un fluorochrome qui présente un changement de fluorescence suite à la liaison dudit agent bioactif potentiel à ladite substance à analyser ciblée.

16. Procédé selon la revendication 12, dans lequel ladite liaison scissile est une liaison photoclivable.

17. Procédé selon la revendication 12, dans lequel ladite liaison scissile est une liaison clivable par une enzyme.

18. Procédé selon la revendication 12, dans lequel lesdites microsphères poreuses sont distribuées sur une surface à des sites discrets.

19. Procédé selon la revendication 13, dans lequel ladite entité identificatrice est un ligand de liaison à l'identificateur qui se lie avec un ligand de liaison à un décodeur de sorte que l'agent bioactif potentiel soit identifié.

20. Procédé selon la revendication 19, dans lequel ledit ligand de liaison à l'identificateur est une sonde d'acide nucléique.

21. Procédé selon la revendication 19, dans lequel ledit ligand de liaison à l'identificateur est une protéine.

22. Procédé selon la revendication 19, dans lequel l'entité identificatrice est une signature optique.

23. Procédé selon la revendication 22, dans lequel ladite signature optique comprend un colorant fluorescent.

24. Procédé selon la revendication 22, dans lequel ladite signature optique comprend deux colorants fluorescents ou plus.

25. Procédé selon la revendication 12, dans lequel au moins deux sous-populations contiennent des quantités différentes du même agent bioactif potentiel.

26. Procédé selon la revendication 12, dans lequel ladite substance à analyser ciblée est une protéine.

27. Procédé selon la revendication 26, dans lequel la protéine est une enzyme.
